# EUROPEAN PATENT SPECIFICATION

(11) **EP 4 380 567 B1**
(45) Date of publication and mention of the grant of the patent: **07.05.2025**
(21) Application number: 22761521.8
(22) Date of filing: 04.08.2022
(51) Int. Cl.: A61K 31/4409, A61K 31/4402, A61K 31/35, A61K 31/421, A61K 31/426, A61P 31/04, A61P 35/00

(54) **DISULFIDE COMPOUNDS AS MEDICAMENTS**
DISULFIDVERBINDUNGEN ALS MEDIKAMENTE
COMPOSÉS DE DISULFIDE EN TANT QUE MÉDICAMENTS

(30) Priority: 04.08.2021 EP 21189723
(43) Date of publication of application: 12.06.2024
(73) Proprietor: Cytacoat AB, 171 64 Solna (SE)
(72) Inventor: WIRSÉN, Per, 78630 Orgeval (FR); TEJBRANT, Jan, 122 42 Enskede (SE); SURKOV, Serhiy, 756 44 Uppsala (SE); RHODES, Alan, Wokingham Berkshire RG41 5GS (GB)
(74) Representative: Novitas Patent AB
(86) International application number: PCT/EP2022/072006
(87) International publication number: WO 2023/012305

(56) References cited:
- WO-A1-2009/123597
- WO-A1-2010/140148
- WO-A1-2015/009699
- WO-A2-2008/052126
- TERNAY A L JR ET AL: "Organosulfur compounds as pre-exposure therapy for threat agents", JOURNAL OF APPLIED TOXICOLOGY, WILEY HEYDEN LTD, GB, vol. 20, no. suppl. 1, 1 January 2000 (2000-01-01), pages S31 - S34, XP009140640, ISSN: 0260-437X, DOI: 10.1002/JAT.692
- SMITH MUNEERAH ET AL: "The cytotoxicity of garlic-related disulphides and thiosulfonates in WHCO1 oesophageal cancer cells is dependent onS-thiolation and not production of ROS", BIOCHIMICA ET BIOPHYSICA ACTA, ELSEVIER, AMSTERDAM, NL, vol. 1860, no. 7, 4 April 2016 (2016-04-04), pages 1439 - 1449, XP029625378, ISSN: 0304-4165, DOI: 10.1016/J.BBAGEN.2016.03.032
- WEI XIANGXU ET AL: "Synthesis and biological evaluation of disulfides as anticancer agents with thioredoxin inhibition", BIOORGANIC CHEMISTRY, vol. 110, 1 May 2021 (2021-05-01), US, pages 104814, XP055877476, ISSN: 0045-2068, DOI: 10.1016/j.bioorg.2021.104814
- CATTEL LUIGI ET AL: "2,3-oxidosqualene cyclase: From azasqualenes to new site-directed inhibitors", LIPIDS, vol. 30, no. 3, 1 March 1995 (1995-03-01), DE, pages 235 - 246, XP055877479, ISSN: 0024-4201, DOI: 10.1007/BF02537827
- YOUNGHWA NA ET AL: "7- N -(Mercaptoalkyl)mitomycins: Implications of Cyclization for Drug Function", JOURNAL OF THE AMERICAN CHEMICAL SOCIETY, vol. 124, no. 17, 1 May 2002 (2002-05-01), pages 4666 - 4677, XP055057053, ISSN: 0002-7863, DOI: 10.1021/ja0124313

## Description

### Technical field

The present invention relates to disulfide compounds for use as medicaments. Specifically, the invention relates to such compounds for use as antimicrobial agents and antimicrobial agents comprising said compounds.

### Background of the invention

Bacteria are ubiquitous. They play an important role in maintaining the environment in which we live. Only a small percentage of the world's bacteria cause infection and disease. These bacterial infections can have a large impact on public health.

Bacteria are unique among the prokaryotes in that so many of them are normal flora that colonize the host without causing infection. Once a person is infected, clinically apparent disease may or may not be seen, and only in a small subset of infections do we see clinically significant disease. Bacterial infections can be transmitted by a variety of mechanisms. In order to be spread, a sufficient number of organisms must survive in the environment and reach a susceptible host. Clearly, measures to prevent infection have a dramatic impact on morbidity and mortality.

Prevention is especially important in this age of increasing antibiotic resistance because treatment can be so difficult to achieve. There are three major principals of control of bacterial infection: eliminate or contain the source of infection, interrupt the chain of transmission, and protect the host against infection or disease. There is a continuous need for the development of new antibacterial agents.

WO2015/009699 discloses *inter alia* that the compound 2-(sec-butyldisulfanyl)pyridine has an antimicrobial activity.

The present inventors have unexpectedly found that a class of disulfide compounds possess antimicrobial activity. Such compounds also offer the potential for wider use as medicaments, as well as for use in the inhibition of unregulated cell growth, such as cancer cell growth.

### Summary of the Invention

In a first aspect, the disclosure provides a disulfide compound of formula (I) 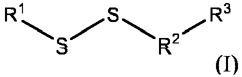 and stereochemically isomeric forms thereof, wherein
R' is a saturated or unsaturated 3 to 20 membered carbocyclic or heterocyclic ring system;
R² is selected from the group consisting of -(CX₂)ₘ where m is 2-1000 and each X is -H, -CH₃, -(CH₂)ₙCH₃ where n is 1 to 1000, -halogen, -OH, O-C₁-C₆ alkyl, or an ether, ester, carbamate, carbonate, amide, amine, urea, or thiol bond and wherein the -(CX₂)ₘ chain is optionally substituted with alkyl, alkenyl, halogen, alkoxy, amine, ether, alcohol or acid groups; -Ar(CH₂)ₙ or -(CH₂)ₙAr where n is 1 to 1000 and Ar is an optionally substituted saturated or unsaturated carbocycle or heterocycle; C₃-C₂₀ cycloalkyl; C₂-C₅₀ alkenyl; and (CH₂CH₂O)ₙ(CH₂)_{P} or (CH(CH₃)CH₂O)ₙ(CH₂)ₚ where n is 1-1000 and p is 0-20; and
R³ is hydrogen or a moiety comprising a functional group selected from the group consisting of alkyl, alkoxy, amine, hydroxyl, carboxyl, imine, thiol, amide, guanidine, acrylamide, acrylate, methacrylate, acetate, allyl, vinyl, carbonyl, azo, nitrile, epoxide, ester, phosphate and sulfate;
and the pharmaceutically acceptable salts thereof for use as a medicament.

The invention is defined by the claims. Any subject-matter beyond the scope of the claims is disclosed for reference or comparison only.

Any reference to a non-patentable method of treatment of the human or animal body by therapy by a certain compound or composition is to be understood as referring to said compound or composition for use in said therapy.

In a second aspect, the invention provides a disulfide compound as claimed for use as an antimicrobial agent. A disulfide compound as hereinbefore defined is disclosed for use in the inhibition of unregulated cell growth.

In a fourth aspect, the invention relates to an antimicrobial agent in the form of a pharmaceutical composition comprising a disulfide compound as defined in the claims.

In a fifth aspect, the invention relates to the non-therapeutic use of a disulfide compound as defined in the claims as an antimicrobial agent.

### Definitions

The term "antimicrobial agent" will be understood to mean an agent which is capable of inhibiting the propagation of microbes, which may be either the inhibition of the propagation by a microbiocidal action (killing the microbes) or by a microbiostatic action (inhibiting the growth of the microbes), for example. It will further be understood to encompass, for example, antibacterial agents, antifungal agents and antiviral agents.

The term "stereochemically isomeric forms" as used herein defines all the possible isomeric forms that the disulfide compounds may possess. Unless otherwise mentioned or indicated, the chemical designation of compounds denotes the mixture of all possible stereochemically isomeric forms, said mixtures containing all diastereomers and enantiomers of the basic molecular structure. The invention also embraces each of the individual isomeric forms of the disulfide compounds and their salts, substantially free, i.e. associated with less than 10%, preferably less than 5%, in particular less than 2% and most preferably less than 1% of the other isomers.

The term "pharmaceutically acceptable" refers to chemical compounds and mixtures thereof that are acceptable to be used in drug products. All excipients used in regulatory approved drug products are pharmaceutically acceptable.

The term "excipient" refers to chemical compounds for use in drug products where said excipients *per* se are not biologically active in the amount present when the drug product is used according to the intention or regulatory approval.

The term "prodrug" means a compound which is inactive in the intended pharmacological action but which can be converted into a pharmacologically active agent by metabolic or physico-chemical transformations.

### Detailed Description of the Invention

### Disulfide compounds

Disclosed are disulfide compounds of formula (I): 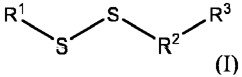 and stereochemically isomeric forms thereof, wherein
R' is a saturated or unsaturated 3 to 20 membered carbocyclic or heterocyclic ring system;
R² is selected from the group consisting of -(CX₂)ₘ where m is 2-1000 and each X is -H, -CH₃, -(CH₂)ₙCH₃ where n is 1 to 1000, -halogen, -OH, O-C₁-C₆ alkyl, or an ether, ester, carbamate, carbonate, amide, amine, urea, or thiol bond and wherein the -(CX₂)ₘ chain is optionally substituted with alkyl, alkenyl, halogen, alkoxy, amine, ether, alcohol or acid groups; -Ar(CH₂)ₙ or -(CH₂)ₙAr where n is 1 to 1000 and Ar is an optionally substituted saturated or unsaturated carbocycle or heterocycle; C₃-C₂₀ cycloalkyl; C₂-C₅₀ alkenyl; and (CH₂CH₂O)ₙ(CH₂)_{P} or (CH(CH₃)CH₂O)ₙ(CH₂)ₚ where n is 1-1000 and p is 0-20; and
R³ is hydrogen or a moiety comprising a functional group selected from the group consisting of alkyl, alkoxy, amine, hydroxyl, carboxyl, imine, thiol, amide, guanidine, acrylamide, acrylate, methacrylate, acetate, allyl, vinyl, carbonyl, azo, nitrile, epoxide, ester, phosphate and sulfate;
and the pharmaceutically acceptable salts thereof

As defined above, R¹ in compounds of formula (I) is a saturated or unsaturated 3 to 20 membered carbocyclic or heterocyclic ring system. The ring system may be monocyclic or multicyclic (e.g. bi-, tri- or tetra-cyclic). Where the ring system is multicyclic, the rings may be fused.

Ring system R¹ may be substituted or unsubstituted. Where the ring system is substituted, possible substituents include C₁-C₈ alkyl groups (e.g. methyl, ethyl), halo groups (e.g. chloro), C₁-C₈ alkoxy groups (e.g. methoxy, ethoxy) and amine groups (e.g. acetamido, amino).

In a preferred embodiment, R¹ is a saturated or unsaturated 4 to 16 membered carbocyclic or heterocyclic ring system, more preferably 5 to 10 membered carbocyclic or heterocyclic ring system, such as a 6 membered carbocyclic or heterocyclic ring system.

Particularly preferred ring systems for R¹ include benzyl, p-nitro-benzyl, o-nitro-benzyl, para-trifluoromethyl-benzyl, 2,4,6-trifluoro-benzyl, 4-Fluoro benzyl, ortho-pyridyl, meta-pyridyl, para-pyridyl, 4-Nitro-pyridin, pyrazine, pyrimidine, quinoxaline, azepin, 1,4-diazepin, quinoline, isoquinoine, purine, pteridine, imidazole, thiazole, benzothiazole, 2-( 5,5-dimethyl-1,3,2-dioxaphosphorinane 2-sulfide), aziridine, tetrazole, siloxane and piperidine.

In the context of the R² moiety, optional substituents on the Ar group may be selected from the group consisting of C₁-C₈ alkyl groups (e.g. methyl, ethyl), C₂-C₂₀ alkenyl groups, ether groups, alcohol groups, acid groups, halo groups (e.g. chloro), C₁-C₈ alkoxy groups (e.g. methoxy, ethoxy) and amine groups
(e.g. acetamido, amino).

In a preferred embodiment, R² is selected from the group consisting of:
- Alkyl chain -(CH₂)ₘ- where m is 2 to 500, optionally substituted with alkyl, alkenyl, halogen, alkoxy, amine, ether, alcohol or acid groups. The alkyl chain may optionally contain ether, ester, carbamate, carbonate, amide, amine, urea, or thiol bonds, thus the alkyl chain may comprise alkyl ether, alkyl ester, alkyl carbamate alkyl carbonate, alkylamide, alkylamine, alkyl urea or alkyl thiol groups;
- Aryl (Ar) group -Ar(CH₂)ₙ- or -(CH₂)ₙAr- where Ar is a saturated or unsaturated carbocycle or heterocycle, optionally substituted with alkyl, alkenyl, halogen, alkoxy, amine, ether, alcohol or acid groups and n is 1 to 500 and wherein said carbocycle or heterocycle may optionally contain ether, ester, carbamate, carbonate, amide, amine, urea, or thiol bonds, thus the carbocycle or heterocycle may comprise alkyl ether, alkyl ester, alkyl carbamate alkyl carbonate, alkylamide, alkylamine, alkyl urea or alkyl thiol groups;
- Cycloalkyl (Cy) group -Cy(CH₂)ₒ- or -(CH₂)ₒCy- where Cy is optionally substituted with alkyl, alkenyl, halogen, alkoxy, amine, ether, alcohol or acid groups and o is 1 to 500 and said cycloalkyl group may optionally contain ether, ester, carbamate, carbonate, amide, amine, urea, or thiol bonds, thus the cycloalkyl group may comprise alkyl ether, alkyl ester, alkyl carbamate alkyl carbonate, alkylamide, alkylamine, alkyl urea or alkyl thiol groups; and
- Ether chain (CH₂CH₂O)ₙ(CH₂)ₚ or (CH(CH₃)CH₂O)ₙ(CH₂)ₚ where n is 1 to 500 and p is 0 to 20.

In another preferred embodiment, R² is selected from the group consisting of:
- Alkyl chain -(CH₂)ₘ- where m is 2 to 100, optionally substituted with alkyl, alkenyl, halogen, alkoxy, amine, ether, alcohol or acid groups. The alkyl chain may optionally contain ether, ester, carbamate, carbonate, amide, amine, urea, or thiol bonds, thus the alkyl chain may comprise alkyl ether, alkyl ester, alkyl carbamate alkyl carbonate, alkylamide, alkylamine, alkyl urea or alkyl thiol groups;
- Aryl (Ar) group -Ar(CH₂)ₙ- or -(CH₂)ₙAr- where Ar is a saturated or unsaturated carbocycle or heterocycle, optionally substituted with alkyl, alkenyl, halogen, alkoxy, amine, ether, alcohol or acid groups and n is 1 to 100 and wherein said carbocycle or heterocycle may optionally contain ether, ester, carbamate, carbonate, amide, amine, urea, or thiol bonds, thus the carbocycle or heterocycle may comprise alkyl ether, alkyl ester, alkyl carbamate alkyl carbonate, alkylamide, alkylamine, alkyl urea or alkyl thiol groups;

- Cycloalkyl (Cy) group -Cy(CH₂)ₒ- or -(CH₂)ₒCy- where Cy is optionally substituted with alkyl, alkenyl, halogen, alkoxy, amine, ether, alcohol or acid groups and o is 1 to 100 and said cycloalkyl group may optionally contain ether, ester, carbamate, carbonate, amide, amine, urea, or thiol bonds, thus the cycloalkyl group may comprise alkyl ether, alkyl ester, alkyl carbamate alkyl carbonate, alkylamide, alkylamine, alkyl urea or alkyl thiol groups; and
- Ether chain (CH₂CH₂O)ₙ(CH₂)ₚ or (CH(CH₃)CH₂O)ₙ(CH₂)ₚ where n is 1 to 250 and p is 0 to 20.

In yet another preferred embodiment, R² is selected from the group consisting of:
- Alkyl chain -(CH₂)ₘ- where m is 2 to 20, optionally substituted with alkyl, alkenyl, halogen, alkoxy, amine, ether, alcohol or acid groups. The alkyl chain may optionally contain ether, ester, carbamate, carbonate, amide, amine, urea, or thiol bonds, thus the alkyl chain may comprise alkyl ether, alkyl ester, alkyl carbamate alkyl carbonate, alkylamide, alkylamine, alkyl urea or alkyl thiol groups;
- Aryl (Ar) group -Ar(CH₂)ₙ- or -(CH₂)ₙAr- where Ar is a saturated or unsaturated carbocycle or heterocycle, optionally substituted with alkyl, alkenyl, halogen, alkoxy, amine, ether, alcohol or acid groups and n is 1 to 20 and wherein said carbocycle or heterocycle may optionally contain ether, ester, carbamate, carbonate, amide, amine, urea, or thiol bonds, thus the carbocycle or heterocycle may comprise alkyl ether, alkyl ester, alkyl carbamate alkyl carbonate, alkylamide, alkylamine, alkyl urea or alkyl thiol groups;
- Cycloalkyl (Cy) group -Cy(CH₂)ₒ- or -(CH₂)ₒCy- where Cy is optionally substituted with alkyl, alkenyl, halogen, alkoxy, amine, ether, alcohol or acid groups and o is 1 to 20 and wherein said cycloalkyl group may optionally contain ether, ester, carbamate, carbonate, amide, amine, urea, or thiol bonds, thus the cycloalkyl group may comprise alkyl ether, alkyl ester, alkyl carbamate alkyl carbonate, alkylamide, alkylamine, alkyl urea or alkyl thiol groups; and
- Ether chain (CH₂CH₂O)ₙ(CH₂)ₚ or (CH(CH₃)CH₂O)ₙ(CH₂)ₚ where n is 1 to 50 and p is 0 to 20.

In a further preferred embodiment, R² is selected from the group consisting of:
- Alkyl chain -(CH₂)ₘ- where m is 2 to 20, optionally substituted with alkyl, alkoxy, amine, ether, alcohol or acid groups. The alkyl chain may optionally contain ether, ester, amide or amine bonds, thus the alkyl chain may comprise alkyl ether, alkyl ester, alkyl amide or alkylamine groups; and
- Ether chain (CH2CH2O)ₙ(CH2)ₚ or (CH(CH₃)CH₂O)ₙ(CH₂)ₚ where n is 1 to 50 and p is 0 to 20.

It is especially preferred if R² is selected from the group consisting of -(CH₂)ₘ- where m is 2-500, more preferably 2-100, even more preferably 2 to 20.

In a particularly preferred embodiment, R² is (CH₂)₂.

As defined above, R³ in compounds of formula (I) is hydrogen or a moiety comprising a functional group selected from the group consisting of alkyl, alkoxy, amine, hydroxyl, carboxyl, imine, thiol, amide, guanidine, acrylamide, acrylate, methacrylate, acetate, allyl, vinyl, carbonyl, azo, nitrile, epoxide, ester, phosphate and sulfate.

In a preferred embodiment, R³ is hydrogen or a moiety comprising a functional group selected from the group consisting of alkyl, alkoxy, amine, hydroxyl, carboxyl, imine, thiol, amide, guanidine, acetate, allyl, vinyl, carbonyl, nitrile, epoxide, ester, phosphate and sulfate.

In another preferred embodiment, R³ is hydrogen or a moiety comprising a functional group selected from the group consisting of alkyl, alkoxy, amine, hydroxyl, carboxyl, imine, amide, guanidine, acetate, allyl, vinyl, ester, phosphate and sulfate.

In yet another preferred embodiment, R³ is hydrogen or a moiety comprising a functional group selected from the group consisting of alkyl, alkoxy, amine, hydroxyl, carboxyl, amide, guanidine, acetate, allyl, vinyl and ester.

In a further preferred embodiment, R³ is hydrogen or a moiety comprising a functional group selected from the group consisting of alkyl, alkoxy (e.g. methoxy or ethoxy), amine, hydroxyl, carboxyl (e.g. COOH), amide, guanidine, acetate, allyl, vinyl and ester.

In yet another preferred embodiment, R³ is hydrogen or a moiety comprising a functional group selected from the group consisting of alkoxy (e.g. methoxy or ethoxy), amine, hydroxyl, carboxyl (e.g. COOH), amide, guanidine, acetate, allyl, vinyl and ester.

Preferably, R³ comprises an amino or carboxyl group.

The amino group may be a primary, secondary, tertiary or quaternary amine.

Particularly preferred embodiments are wherein R³ is NR₂, wherein R is H or CH₃, or wherein R³ is COOH.

Whenever compounds of formula (I) are in the form of salts, R³ may be (NR₃)⁺, wherein R is H or CH₃ (methyl) or CH₂CH₃ (ethyl).

In a preferred embodiment, the disulfide compound is a compound of formula (II) 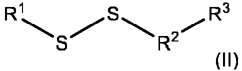 and stereochemically isomeric forms thereof, wherein
R¹ is a saturated or unsaturated 3 to 20 membered heterocyclic ring system;
R² is selected from the group consisting of:
   - Alkyl chain -(CH₂)ₘ- where m is 2 to 20, optionally substituted with alkyl, alkenyl, halogen, alkoxy, amine, ether, alcohol or acid groups. The alkyl chain may optionally contain ether, ester, carbamate, carbonate, amide, amine, urea, or thiol bonds;
   - Aryl (Ar) group -Ar(CH₂)ₙ- or -(CH₂)ₙAr- where Ar is a saturated or unsaturated carbocycle or heterocycle, optionally substituted with alkyl, alkenyl, halogen, alkoxy, amine, ether, alcohol or acid groups and n is 1 to 20 and wherein said carbocycle or heterocycle may optionally contain ether, ester, carbamate, carbonate, amide, amine, urea, or thiol bonds;
   - Cycloalkyl (Cy) group -Cy(CH₂)ₒ- or -(CH₂)ₒCy- where Cy is optionally substituted with alkyl, alkenyl, halogen, alkoxy, amine, ether, alcohol or acid groups and o is 1 to 20 and wherein said cycloalkyl group may optionally contain ether, ester, carbamate, carbonate, amide, amine, urea, or thiol bonds; and
   - Ether chain (CH2CH2O)ₙ(CH2)ₚ or (CH(CH₃)CH₂O)ₙ(CH₂)ₚ where n is 1 to 50 and p is 0 to 20; and
R³ is hydrogen or a moiety comprising a functional group selected from the group consisting of alkyl, alkoxy, amine, hydroxyl, carboxyl, amide, guanidine, acetate, allyl, vinyl and ester;
and the pharmaceutically acceptable salt thereof.

In this embodiment, the R¹ heterocyclic ring system comprises one or more heteroatoms, such as nitrogen, oxygen or sulfur. Preferably, the one or more heteroatoms is nitrogen.

The heterocyclic ring system may be saturated or unsaturated carbon ring system and may be monocyclic or multicyclic (e.g. bi-, tri- or tetra-cyclic). Where the ring system is multicyclic, the rings may be fused.

Heterocyclic ring system R¹ may be substituted or unsubstituted. Where the ring system is substituted, possible substituents include C₁-C₈ alkyl groups (e.g. methyl, ethyl), halo groups (e.g. chloro), C₁-C₈ alkoxy groups (e.g. methoxy, ethoxy) and amine groups (e.g. acetamido, amino).

In a preferred embodiment, R¹ is C₄-C₁₆ heterocyclic ring system, more preferably C₅ to C₁₀, such as a C₆ heterocyclic ring system.

Examples of preferred heterocyclic ring systems include pyridine and pyrimidine.

The -S-S- bridge in compounds of formula (II) may be at the 2 (ortho) and/or 4 (para) position(s) relative to heteroatom.

In formula (II), R³ is preferably hydrogen or a moiety comprising a functional group selected from the group consisting of alkoxy, amine, hydroxyl, carboxyl, amide, guanidine, acetate, allyl, vinyl and ester

In a further preferred embodiment, the disulfide compound is a compound of formula (III) 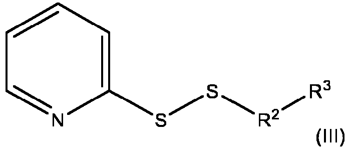 and stereochemically isomeric forms thereof, wherein
R² is selected from the group consisting of:
   - Alkyl chain -(CH₂)ₘ- where m is 2 to 20, optionally substituted with alkyl, alkoxy, amine, ether, alcohol or acid groups. The alkyl chain may optionally contain ether, ester, amide or amine bonds; and
   - Ether chain (CH₂CH₂O)ₙ(CH2)ₚ or (CH(CH₃)CH₂O)ₙ(CH₂)ₚ where n is 1 to 50 and p is 0 to 20 ;and
R³ is hydrogen or a moiety comprising a functional group selected from the group consisting of alkyl, alkoxy (e.g. methoxy or ethoxy), amine, hydroxyl, carboxyl (e.g. COOH), amide, guanidine, acetate, allyl, vinyl and ester;
and the pharmaceutically acceptable salts and prodrugs thereof.

In formula (III), R³ is preferably hydrogen or a moiety comprising a functional group selected from the group consisting of alkoxy (e.g. methoxy or ethoxy), amine, hydroxyl, carboxyl (e.g. COOH), amide, guanidine, acetate, allyl, vinyl and ester.

In a further preferred embodiment, the disulfide compound is a compound of formula (IV) 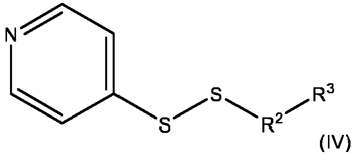 and stereochemically isomeric forms thereof, wherein
R² is selected from the group consisting of -(CH₂)ₘ- where m is 2-500, more preferably 2-100, even more preferably 2 to 20; and
R³ comprises an amino or carboxyl group; and the pharmaceutically acceptable salts thereof.

Specific examples of the disulfide compounds of the disclosure are:

| Compound | Structure |
|---|---|
| 2-(Pyridyldithio)ethylamin e (hydrochloride) (PDEA) | 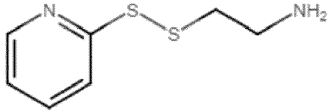 |
| PDEA analogue with C₈ linker | 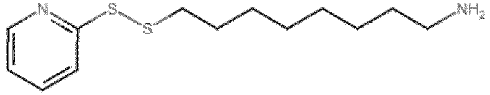 |
| PDEA (para position) | 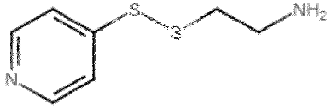 |
| PDEA analogue (para position) with C₈ linker | 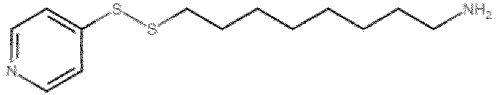 |
| PDEA analogue with secondary amine (methyl) tail | 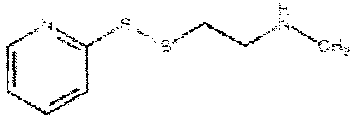 |
| PDEA analogue with tertiary amine (methyl) tail | 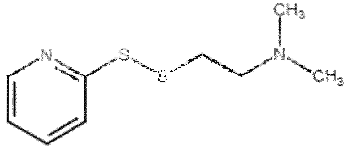 |
| PDEA analogue (para position) with tertiary amine (methyl) tail | 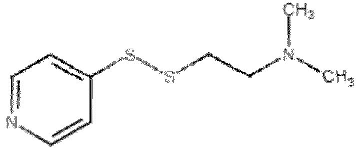 |
| PDEA analogue with tertiary amine (ethyl) tail | 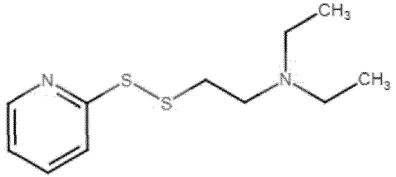 |
| PDEA analogue with quaternary amine (methyl) tail | 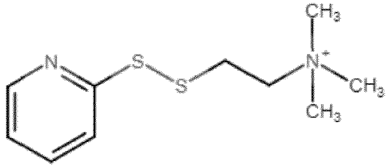 |
| PDEA analogue (para position) with quaternary amine (methyl) tail | 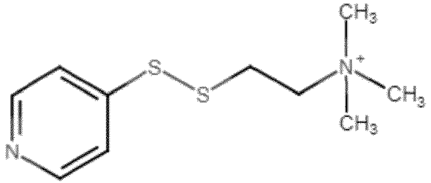 |
| PDEA analogue with quaternary amine (ethyl) tail | 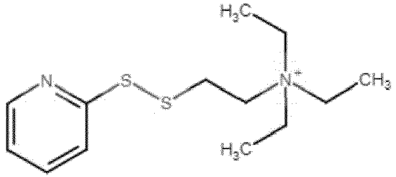 |
| PDEA analogue with quaternary amine (butyl) tail | 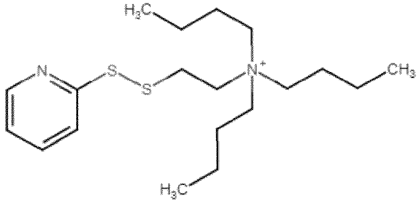 |
| PDEA Analogue with guanidine tail | 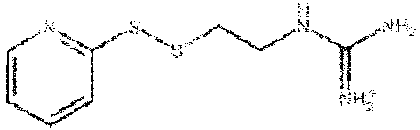 |
| PDEA analogue with alkyl (propyl) tail | 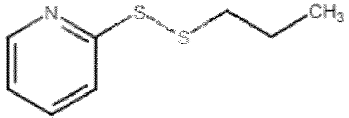 |
| PDEA analogue (para position) with alkyl (propyl) tail | 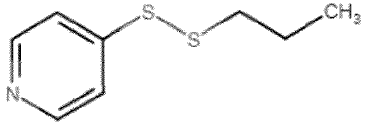 |
| PDEA analogue with alkyl (hexyl) tail | 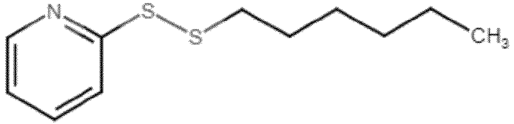 |
| PDEA analogue with alkyl (dodecyl) tail | 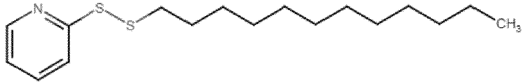 |
| 2-(2-Pyridinyldithio)ethanol | 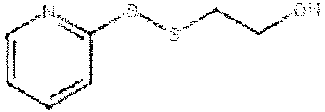 |
| PDEA analogue with primary alcohol tail | |
| PDEA analogue (para position) with primary alcohol tail | 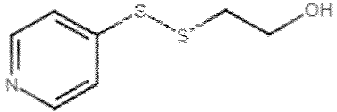 |
| PDEA analogue with primary alcohol tail and C₈ linker | 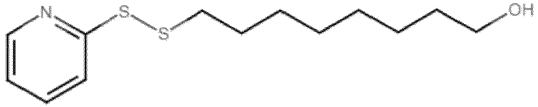 |
| PDEA analogue with diol tail | 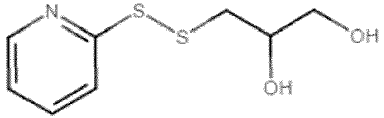 |
| 3-(2-Pyridyldithio)propanoic acid PDEA analogue with carboxylic acid tail | 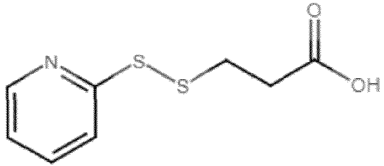 |
| PDEA analogue with C₈ linker and carboxylic acid tail | 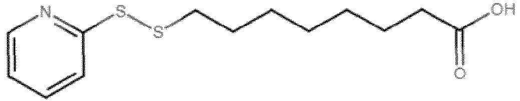 |
| PDEA analogue (para position) with carboxylic acid tail | 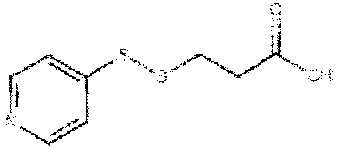 |
| PDEA analogue with acetate tail | 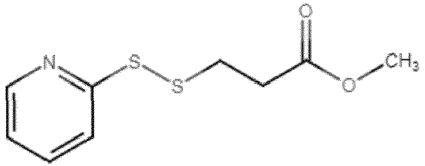 |
| PDEA analogue with secondary alcohol tail and hindered disulfide bond | 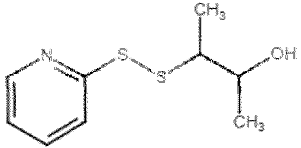 |
| PDEA analogue with primary alcohol tail and hindered disulfide bond | 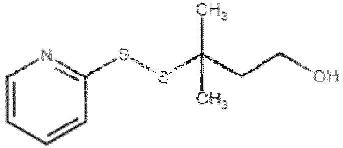 |
| PDEA analogue with alkoxy (methoxy) tail and PEG₂ chain | 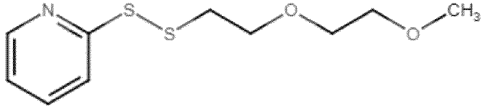 |
| (2-Pyridyldithio)-PEG3-OMe | 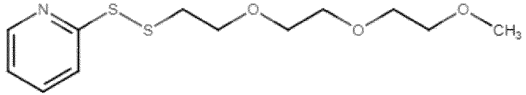 |
| PDEA analogue with alkoxy (methoxy) tail and PEG₃ chain | |
| S-(2-Glycylamidoethyl)dithio-2-pyridine | 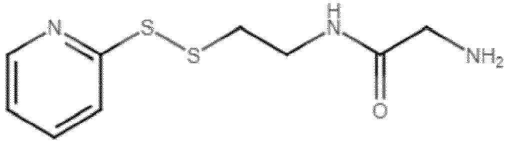 |
| PDEA analogue with acrylamide tail | 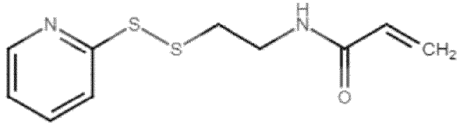 |
| 2-(2-Pyridinyldithio)ethyl acrylate | 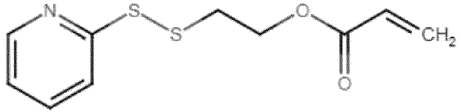 |
| PDEA analogue with acrylate tail | |
| 2-(2-Pyridinyldithio)ethyl methacrylate | 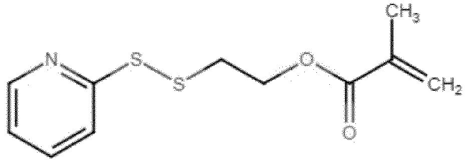 |
| PDEA analogue with methacrylate tail | |
| PDEA analogue with allyl tail | 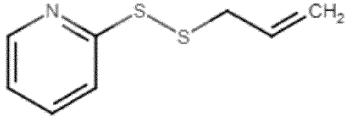 |
| PDEA analogue with cysteine tail | 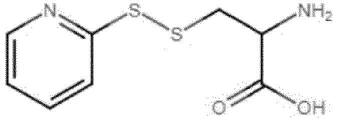 |
| PDEA analogue with N-acetyl cysteine tail | 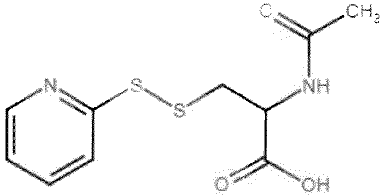 |
| PDEA analogue with thiophenethiol ring instead of pyridyl ring | 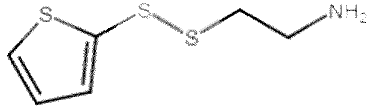 |
| PDEA analogue with 2-mercaptothiazole ring instead of pyridyl ring | 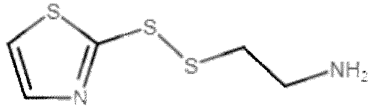 |
| PDEA analogue with oxazole-2-thiol ring instead of pyridyl ring | 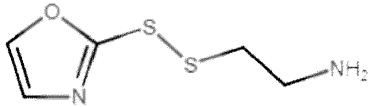 |
| PDEA analogue with oxane-3-thiol ring instead of pyridyl ring | 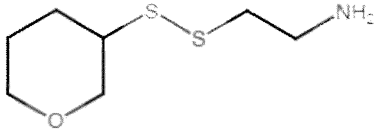 |

A particularly preferred group of compounds of the disclosure are:

| Compound | Structure |
|---|---|
| 2-(Pyridyldithio)ethylamin e (hydrochloride) (PDEA) | 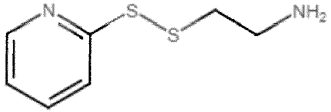 |
| PDEA analogue with C₈ linker | 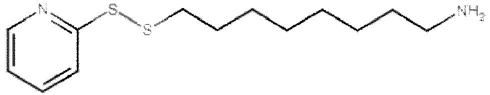 |
| PDEA (para position) | 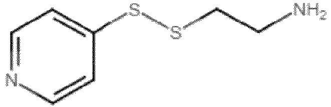 |
| PDEA analogue (para position) with C₈ linker | 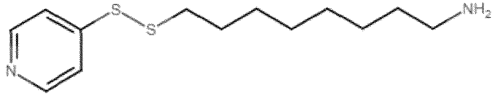 |
| PDEA analogue with secondary amine (methyl) tail | 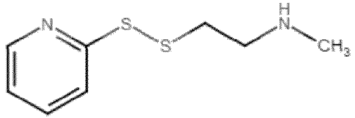 |
| PDEA analogue with tertiary amine (methyl) tail | 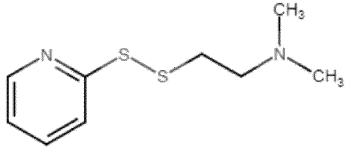 |
| PDEA analogue (para position) with tertiary amine (methyl) tail | 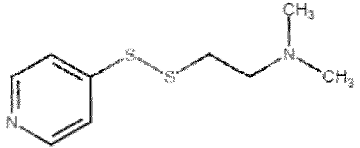 |
| PDEA analogue with tertiary amine (ethyl) tail | 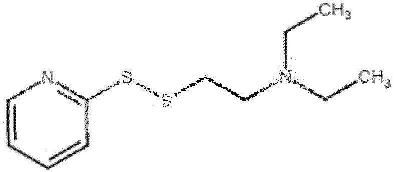 |
| PDEA analogue with quaternary amine (methyl) tail | 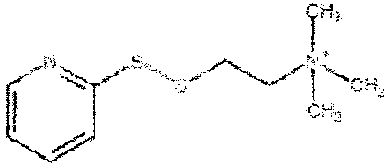 |
| PDEA analogue (para position) with quaternary amine (methyl) tail | 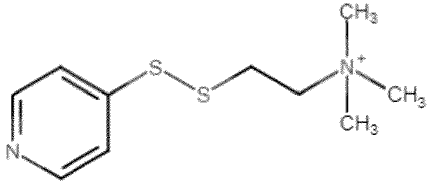 |
| PDEA analogue with quaternary amine (ethyl) tail | 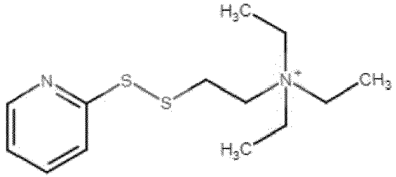 |
| PDEA analogue with quaternary amine (butyl) tail | 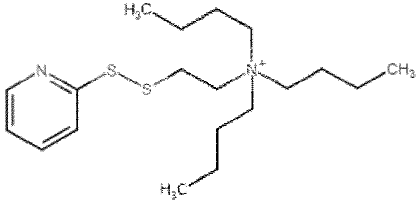 |
| PDEA Analogue with guanidine tail | 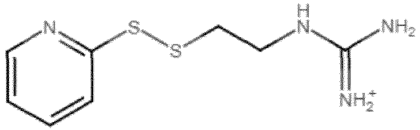 |
| 2-(2-Pyridinyldithio)ethanol | 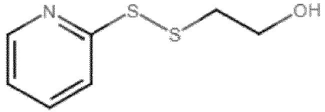 |
| PDEA analogue with primary alcohol tail | |
| PDEA analogue (para position) with primary alcohol tail | 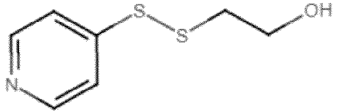 |
| PDEA analogue with primary alcohol tail and C₈ linker | 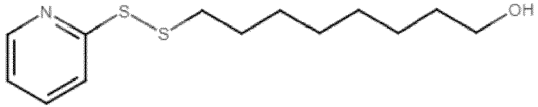 |
| PDEA analogue with diol tail | 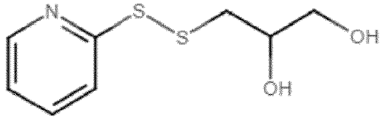 |
| 3-(2-Pyridyldithio)propanoic acid PDEA analogue with carboxylic acid tail | 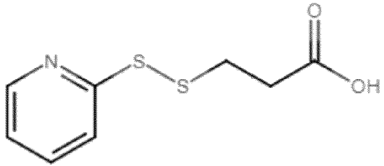 |
| PDEA analogue with C₈ linker and carboxylic acid tail | 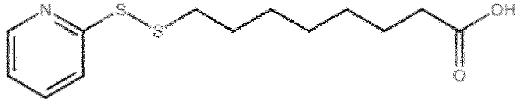 |
| PDEA analogue (para position) with carboxylic acid tail | 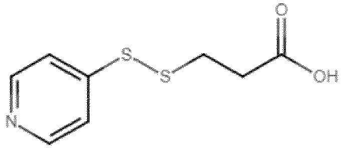 |
| PDEA analogue with acetate tail | 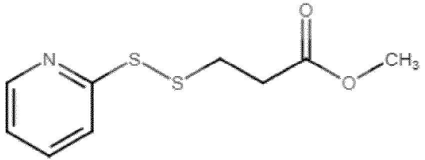 |
| PDEA analogue with secondary alcohol tail and hindered disulfide bond | 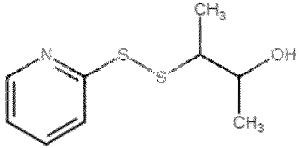 |
| PDEA analogue with primary alcohol tail and hindered disulfide bond | 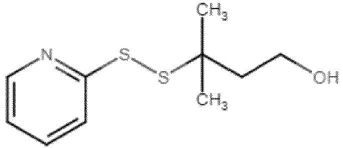 |
| PDEA analogue with alkoxy (methoxy) tail and PEG₂ chain | 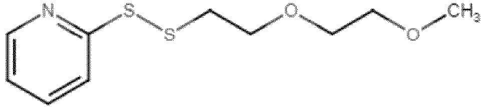 |
| (2-Pyridyldithio)-PEG3-OMe PDEA analogue with alkoxy (methoxy) tail and PEG₃ chain | 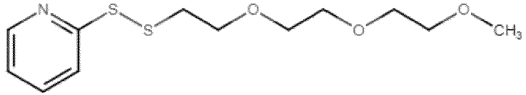 |
| S-(2-Glycylamidoethyl)dithio-2-pyridine | 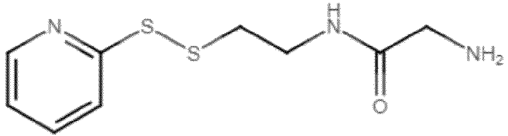 |
| PDEA analogue with acrylamide tail | 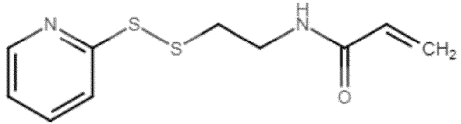 |
| 2-(2-Pyridinyldithio)ethyl acrylate PDEA analogue with acrylate tail | 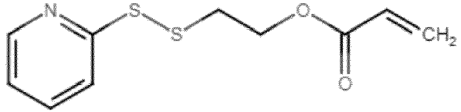 |
| 2-(2-Pyridinyldithio)ethyl methacrylate | 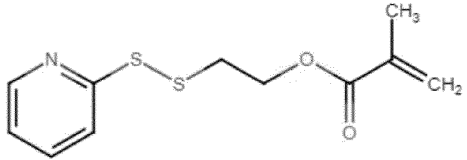 |
| PDEA analogue with methacrylate tail | |
| PDEA analogue with allyl tail | 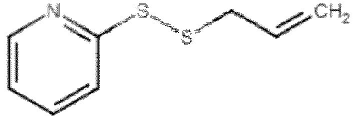 |
| PDEA analogue with cysteine tail | 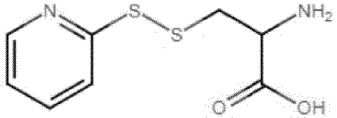 |
| PDEA analogue with N-acetyl cysteine tail | 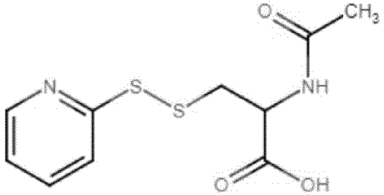 |
| PDEA analogue with thiophenethiol ring instead of pyridyl ring | 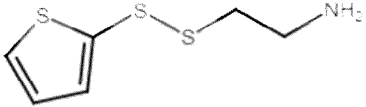 |
| PDEA analogue with 2-mercaptothiazole ring instead of pyridyl ring | 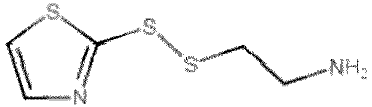 |
| PDEA analogue with oxazole-2-thiol ring instead of pyridyl ring | 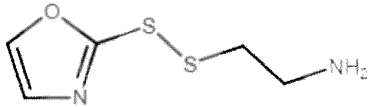 |
| PDEA analogue with oxane-3-thiol ring instead of pyridyl ring | 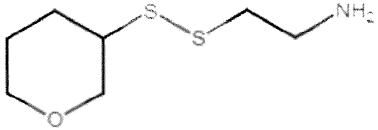 |

The disulfide compounds of the invention are:

| Compound | Structure |
|---|---|
| 2-(Pyridyldithio)ethylamine (hydrochloride) (PDEA) | 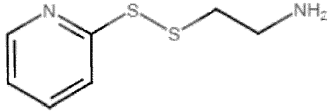 |
| PDEA (para position) | 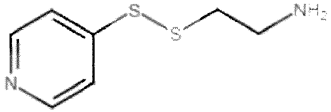 |
| PDEA analogue with quaternary amine (methyl) tail | 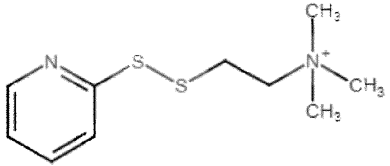 |
| 3-(2-Pyridyldithio)propanoic acid | 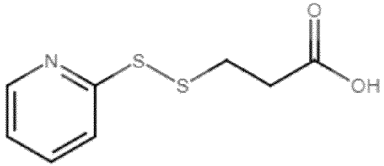 |
| PDEA analogue with carboxylic acid tail | |

The disulfide compound may be in the form of a single enantiomer, or a racemic mixture.

The pharmaceutically acceptable salts are not particularly limited, and examples thereof include inorganic acid salts, organic acid salts, inorganic basic salts, organic basic salts, and acidic or basic amino acid salts. The salts may be native or present together with one or more crystal water molecules.

Particular examples of pharmaceutically acceptable salts include hydrochloride, hydrobromide, sulfate, phosphate, acetate, propoinate, lactate, mesylate, maleate, malate, succinate, tartrate, citrate, fumarate, bensoate, polyacrylate (with e.g. Mw=500-500000), amino acids, ammonium, sodium, potassium, calcium, iron and magnesium.

The compounds according to the invention can generally be prepared by a succession of steps, each of which is known to the skilled person.

### Compositions and Uses

The disulfide compounds as hereinbefore defined may be employed as the compounds *per se* (or the salts thereof) or may be present within a pharmaceutical composition. Thus, the present invention further relates to pharmaceutical compositions comprising a disulfide compound as hereinbefore defined and one or more pharmaceutically acceptable carriers, diluents or excipients. Such carriers, diluents and excipients are well known in the art.

Excipients used in the pharmaceutical compositions of the present invention will vary depending on the nature of the composition. Excipients for suspensions are, in addition to water, typically selected among sodium chloride or other physiologically acceptable salts, sugars, surfactant, antioxidants aromas, sweeteners and pH modifiers. Typically oral capsules are capsules prepared from gelatin or hydroxypropyl methyl cellulose (HPMC). Typical excipients in such capsules might include lactose, microcrystalline cellulose and inorganic salts. Typically tablets can be tablets that disintegrate immediately, controlled release tablets and sustained release tablets. Typical excipients in tablets include for example corn starch, lactose, glucose, microcrystalline cellulose, croscarmellose sodium and magnesium stearate.

The pharmaceutical composition can be in any pharmaceutically acceptable formulation depending on route of administration. For oral administration aqueous suspension, tablet and capsules are the most preferred formulations, for dermal use creams and ointments are preferred pharmaceutical formulations. Regarding injections, the most preferred injections are intravenous injections, intramuscular injections and subcutaneous injections. The injection formulations are typically in the form of sterile aqueous suspensions. Pulmonary formulations according to the present invention in the form of dry powder for inhalation, are typically in the form of single doses or multi dose, or in the form of suspension of particles. Eye products are typically sterile aqueous suspensions of particles, while typical compositions for administration into the nose can be dry particles or an aqueous suspension.

In one embodiment, the pharmaceutical compositions as hereinbefore described are formulation for parenteral administration, e.g. injection or infusion.

In one particularly preferred embodiment of the invention, the pharmaceutical compositions as hereinbefore defined are formulated for oral administration, e.g. as tablets, capsules or a suspension.

The compounds or compositions thereof are preferably administered in a therapeutically effective amount. A "therapeutically effective amount" refers to an amount necessary to induce the desired therapeutic effect. Depending on the mode of administration, the pharmaceutical composition will typically comprise from 0.05 to 99 % by weight, preferably from 0.1 to 70 % by weight, more preferably from 0.1 to 50 % by weight of the active ingredient (i.e. the disulfide compound), and, from 1 to 99.95 % by weight, preferably from 30 to 99.9 % by weight, more preferably from 50 to 99.9 % by weight of a pharmaceutically acceptable carrier, all percentages being based on the total weight of the composition.

Any route of administration may be used to deliver the compounds to the subject. Suitable administration routes include intramuscular injection, transdermal administration, inhalation, topical application, oral administration, rectal or vaginal administration and parenteral administration (e.g. intravenous, peritoneal, intraarterial or subcutaneous). The preferable routes of administration are oral, topical and parenteral.

For oral administration, aqueous suspension, tablet and capsules are the most preferred formulations, for dermal use creams and ointments are preferred pharmaceutical formulations. Regarding injections, the most preferred injections are intravenous injections, intramuscular injections and subcutaneous injections. The injection formulations are typically in the form of sterile aqueous suspensions. Pulmonary formulations according to the present invention in the form of dry powder for inhalation, are typically in the form of single doses or multi dose, or in the form of suspension of particles. Eye products are typically sterile aqueous suspensions of particles, while typical compositions for administration into the nose can be dry particles or an aqueous suspension.

The exact dosage and frequency of administration depends on the particular disulfide compound used, and the desired application. Furthermore, it is evident that said effective daily amount may be lowered or increased depending on the response of the treated subject and/or depending on the evaluation of the physician prescribing the compounds according to the instant invention.

The present invention relates to disulfide compounds as hereinbefore defined for use as a medicament.

In a further embodiment, the present invention relates to the disulfide compounds according to the current invention for use as antimicrobial agents.

In yet a further embodiment, the present invention relates to an antimicrobial agent comprising a disulfide compound as hereinbefore defined.

The "antimicrobial agent" may preferably be an antibacterial agent, an antiviral agent or an antifungal agent.

The antimicrobial agent may be suitable for use in treating a human or animal subject or for use on devices or products that come into contact with potentially harmful microorganisms.

The antimicrobial agent may be employed in vitro or in vivo.

Thus, the invention also relates to the use of a disulfide compound as hereinbefore defined as an antimicrobial agent.

In one preferred embodiment of the invention, the antimicrobial agent as hereinbefore described is an antibacterial agent.

Examples of microorganisms, for which the invention may be used to prevent growth and/or proliferation of, are anaerobic and aerobic bacteria that encompass both different Gram-positive bacteria chosen from, but not limited to, different species of Staphylococci, such as S. aureus, Methicillin-resistant S. aureus (MRSA), S. epidermidis and other coagulase-negative staphylococci, S. saphrophyticus, Enterococcus spp, Nesseriae (Meningococci, Gonococci), Streptococci (Viridans, agalactiae, pyogenes, hemolytic and non-hemolytic, group B and D, S. pneumoniae), Chlostridia (perfringens, botulinum), Bacillus megaterium, as well as different Gram-negative species chosen from, but not limited to, different Enterobacter spp, Escherichia coli, extended spectrum beta-lactamase (ESBL) producing E. coli, Klebsiella spp, Proteus, Campylobacter, Yersinia, Shigella, Salmonella, Haemophilus (influenzae), Bacteriodes (fragilis, bivius), Pseudomonas (aeruginosa, cepacia), Legionella (pneumophilia), Neisseria meningitidis, Acinetobacter baumannii, as well as viruses chosen from, but not limited to, coronaviruses, Sars-Cov-2, Influenza A, Influenza B, Respiratory syncytial virus (RSV), Rhinovirus and Rotavirus. Also included are different mycoplasma species and Candida species and different fungi, such as Candida spp, C. tropicales, C. parapsilosis, Cryptococcus neoformans, Aspergillus fumigatus, Tricosporun, Blastoschizomyces, Stenotrophomonas maltophilia, Malassezia, Bukholderia cepafia, Aspergillus.

Thus, in a preferable embodiment, the invention relates to the disulfide compounds as hereinbefore defined for use as an antimicrobial agent against bacteria selected from the group consisting of gram positive Cocci, gram negative Cocci, gram positive Bacilli, gram negative Bacilli, mycobacteria, spirochetes, chlamydiaceae and mycoplasmataceae or fungi selected from the group consisting of candida spp. and candida albicans.

In this context, examples of gram positive Cocci include staphylococcus, streptococcus and enterococci. Examples of gram negative Cocci include Neisseria. Examples of gram positive Bacilli include spore forming and non-spore forming Bacilli. Examples of gram negative Bacilli include enterics, respiratory bacilli and zoonotic bacilli.

A further embodiment of the present invention discloses the disulfide compounds for use in the inhibition of unregulated cell growth, such as cancer cells and / or cancer cells having significant renewal potential, such as cancer stem cells.

Thus, a preferred embodiment of the invention relates to the disulfide compounds of the invention for use in the treatment of cancer. The cancer (cancer cells) may be cells of the following types of cancer: breast cancer, prostate cancer, brain cancer, blood cancer, bone marrow cancer, liver cancer, pancreas cancer, kidney cancer, colon cancer, ovary cancer, lung cancer, testicle cancer, penis cancer, thyroid cancer, parathyroid cancer, pituitary cancer, thymus cancer, retina cancer, uvea cancer, conjunctiva cancer, spleen cancer, head cancer, neck cancer, trachea cancer, gall bladder cancer, rectum cancer, salivary gland cancer, adrenal gland cancer, throat cancer, esophagus cancer, lymph nodes cancer, sweat glands cancer, sebaceous glands cancer, muscle cancer, heart cancer, and stomach cancer. A particularly suitable group of cancers are bladder cancer, prostate cancer, breast cancer, colon cancer, rectal cancer, endometrial cancer, kidney cancer, leukemia, liver and Intrahepatic Bile Duct, lung cancer, non-Hodgkin Lymphoma, pancreatic cancer and thyroid cancer.

### Combination Therapy

The invention also relates to a pharmaceutical composition comprising a disulfide compound according to the invention and one or more other antibiotics as well as to such a composition for use as a medicament and more specifically for use as an antimicrobial agent, such as an antibacterial agent.

The present invention also relates to a product comprising a disulfide compound according to the present invention, a pharmaceutically acceptable salt thereof or prodrug thereof, and one or more other antibiotics as a combined preparation for simultaneous, separate or sequential use as a medicament as well as more specifically for simultaneous, separate or sequential use as an antimicrobial agent. The different drugs of such a combination or product may be combined in a single preparation together with pharmaceutically acceptable carriers or diluents, or they may each be present in a separate preparation together with pharmaceutically acceptable carriers or diluents.

In this embodiment, a particularly preferable class of antibiotics which may be employed as the one or more other antibiotics is aminoglycosides, such as fradiomycin or gentamycin.

### Description of Figures

Figure 1: % cell survival (T-24 cells) vs PDEA concentration (µM)
Figure 2: % cell survival (5637 cells) vs PDEA concentration (µM)

The invention will now be described with reference to the following examples.

### Examples

### Anti-bacterial testing

### Minimum inhibitory concentrations (MICs) protocol

Day 1: Preparation of Test inoculum/bacteria
   1. Using a sterile inoculating loop, transfer bacteria/Candida albicans from frozen stock culture and streak them on a fresh LA plate.
   2. Incubate the plate at 37 °C overnight (o/n) (18-24 h).
Day 2
   1. Prepare bacterial culture by adding 20 ml Mueller Hinton broth (*) to a 250-ml Erlenmeyer flask with sidearm.
   2. Using a sterile inoculating loop, pick approximately 10 colonies from the o/n bacterial culture and transfer bacteria to the flask.
   3. Incubate the E-flask with bacteria on a rotating shaker at 37 °C until OD590 = 0.40. This OD should equal an approximate bacterial count of 1x10⁸ cfu/ml (**).
      - This is measured using a colorimeter (or spectrophotometer). Set reference and press R (= reference) first, then measure the bacterial culture by pressing T (= test).
   4. At OD590 0.40, serially dilute the bacterial suspension 1:10 000 in Mueller Hinton broth to obtain a bacterial target concentration of 1x10⁴ cfu/ml. Determine the initial inoculation by serially diluting 0.1 ml in 0.9 ml blanks of sterile PBS and plating 0.1 ml aliquots to LA plates, preferably.
   5. Different concentrations of the tested compound are prepared in 0.6-0.8 ml of the final dilution of bacterial suspension and placed in 2-ml test tubes.
   6. Incubate test tubes in an orbital shaker incubator at 37°C for 24h.
Day 3
   7. Analyze visible growth, i.e. "Foggy" or "Clear". MIC is being defined as the interval between the highest "Foggy" and the lowest "Clear" concentration of the compound.
(*) When testing Candida albicans, Yeast extract Peptone Dextrose (YPD) broth is being used.
(**) For Candida albicans it is equal to 1x10⁷

### Example 1

### Synthesis of 2-(Pyridyldithio)ethylamine (hydrochloride) (PDEA) (CAS: 106139-15-5)

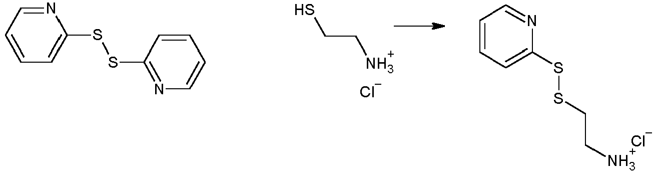

2-Aminoethanethiol hydrochloride (2.00 g, 17.6 mmol) was dissolved in methanol (3 ml) and acetic acid (1 ml). To the solution was added 2,2-dithiodipyridine (5.00 g, 22.7 mmol) dissolved in methanol (20 ml). The mixture was stirred at 22°C overnight and then poured into dry diethyl ether (200 ml). A white precipitate formed that was collected by filtration to give 2.826 g, 12.68 mmol in 72 % yield.

Different concentrations of PDEA (Example 1) in Mueller Hinton medium were prepared. Bacteria were inoculated to a concentration of 20 000 CFU/ml and incubated at 37°C for 24 hours. Minimum inhibitory concentrations (MICs) were determined by analyzing visible growth, i.e., readouts were "Clear" and "Foggy".

### Escherichia coli CFT073

| Conc., mM | 0 | 0.01 | 0.025 | 0.05 | 0.1 |
|---|---|---|---|---|---|
| | Foggy | Foggy | Foggy | Foggy | Clear |
| | Foggy | Foggy | Foggy | Foggy | Clear |
| | Foggy | Foggy | Foggy | Foggy | Clear |

Result: MIC- **0.05-0.1mM**

### Klebsiella pneumoniae AO15200

| Conc., mM | 0 | 0.01 | 0.025 | 0.05 | 0.1 |
|---|---|---|---|---|---|
| | Foggy | Foggy | Foggy | Foggy | Clear |
| | Foggy | Foggy | Foggy | Foggy | Clear |
| | Foggy | Foggy | Foggy | Foggy | Clear |

Result: MIC- **0.05-0.1mM**

### Enteroccocus faecium #11 (KI, clinical isolate)

| Conc., mM | 0 | 0.01 | 0.025 | 0.05 | 0.1 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.025-0.05mM**

### Enteroccocus faecalis #28 (KI, clinical isolate)

| Conc., mM | 0 | 0.05 | 0.1 | 0.2 | 0.4 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.1-0.2mM**

### Staphylococcus epidermidis Se19 (clinical isolate, Jan Ingemar Falk)

| Conc., mM | 0 | 0.01 | 0.025 | 0.05 | 0.1 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.025-0.05mM**

### Staphylococcus aureus B5381

| Conc., mM | 0 | 0.05 | 0.1 | 0.2 | 0.4 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.1-0.2mM**

### Pseudomonas aeruginosa PA02

| Conc., mM | 0 | 0.05 | 0.1 | 0.2 | 0.4 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.1-0.2mM**

Different concentrations of PDEA were also prepared in Yeast extract Peptone Dextrose (YPD) broth. Candida albicans were inoculated to a concentration of 20 000 CFU/ml and incubated at 37°C for 24 hours. Readouts were "Clear" and "Foggy".

| Conc., mM | 0 | 0.01 | 0.025 | 0.05 | 0.1 | 0.2 | 0.4 | 0.8 | 1.6 |
|---|---|---|---|---|---|---|---|---|---|
| | Foggy | Foggy | Foggy | Foggy | Foggy | Foggy | Foggy | Clear | Clear |
| | Foggy | Foggy | Foggy | Foggy | Foggy | Foggy | Foggy | Clear | Clear |
| | Foggy | Foggy | Foggy | Foggy | Foggy | Foggy | Foggy | Clear | Clear |

Result: MIC-**0.4-0.8 mM**

### Example 2

### Synthesis of 2-(2-Pyridyldisulanyl)ethan-(trimethylammonium) chloride

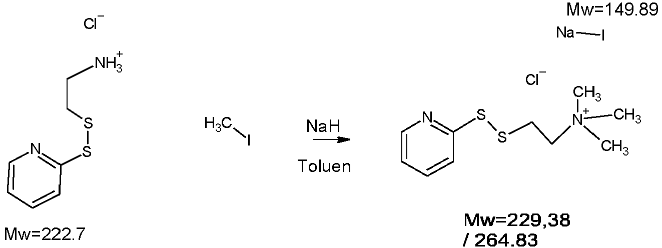

To a suspension of PDEA (0.5 g, 2.25 mmol) in toluene (10 ml), NaH (98 %, 190 mg, 7.52 mmol) was added. Methyl iodide (7 mmol, 0.99 g, 436 µl) was added and the mixture was stirred at 22°C. An aliquot (300 µl) of DMF was added after 1 h and the mixture started to form small bubbles. The mixture was stirred overnight and a white solid formed that was collected by filtration. The solids were washed with diethyl ether to give (1.32 g as the x Cl⁻ and x 3Nal salt theoretically 0.605 g product and 3 eq Nal 1.124 g, total theoretical weight 1.729 g, 76 %).

Different concentrations of the quaternary ammonium PDEA (Example 2) in Mueller Hinton medium were prepared. Bacteria were inoculated to a concentration of 20 000 CFU/ml and incubated at 37°C for 24 hours. Minimum inhibitory concentrations (MICs) were determined by analyzing visible growth, i.e., readouts were "Clear" and "Foggy".

### Escherichia coli CFT073

| Conc., mM | 0 | 0.11 | 0.225 | 0.55 | 1.125 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.225-0.55mM**

### Klebsiella pneumoniae AO15200

| Conc., mM | 0 | 0.11 | 0.225 | 0.55 | 1.125 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.225-0.55mM**

### Enterococcus faecium #11 (KI, clinical isolate)

| Conc., mM | 0 | 0.025 | 0.055 | 0.11 | 0.225 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.055-0.11mM**

### Enterococcus faecalis #28 (KI, clinical isolate)

| Conc., mM | 0 | 0.11 | 0.225 | 0.55 | 1.125 |
|---|---|---|---|---|---|
| | Foggy | Foggy | Clear | Clear | Clear |
| | Foggy | Foggy | Clear | Clear | Clear |
| | Foggy | Foggy | Clear | Clear | Clear |

Result: MIC- **0.11-0.225mM**

### Staphylococcus epidermidis Se19 (clinical isolate, Jan Ingemar Falk)

| Conc., mM | 0 | 0.025 | 0.055 | 0.11 | 0.225 |
|---|---|---|---|---|---|
| | Foggy | Foggy | Foggy | Clear | Clear |
| | Foggy | Foggy | Foggy | Clear | Clear |
| | Foggy | Foggy | Foggy | Clear | Clear |

Result: MIC- **0.055-0.11mM**

### Staphylococcus aureus B5381

| Conc., mM | 0 | 0.11 | 0.225 | 0.55 | 1.125 |
|---|---|---|---|---|---|
| | Foggy | Foggy | Foggy | Foggy | Clear |
| | Foggy | Foggy | Foggy | Foggy | Clear |
| | Foggy | Foggy | Foggy | Foggy | Clear |

Result: MIC- **0.55-1.125mM**

### Pseudomonas aeruginosa PA02

| Conc., mM | 0 | 0.11 | 0.225 | 0.55 | 1.125 |
|---|---|---|---|---|---|
| | Foggy | Foggy | Foggy | Foggy | Clear |
| | Foggy | Foggy | Foggy | Foggy | Clear |
| | Foggy | Foggy | Foggy | Foggy | Clear |

Result: MIC- **0.55-1.125mM**

Different concentrations of the quaternary ammonium PDEA (Example 2) were also prepared in Yeast extract Peptone Dextrose (YPD) broth. Candida albicans were inoculated to a concentration of 20 000 CFU/ml and incubated at 37°C for 24 hours. Readouts were "Clear" and "Foggy".

| PDEA conc., mM | 0 | 0.11 | 0.225 | 0.55 | 1.1 | 2.2 | 4.4 |
|---|---|---|---|---|---|---|---|
| | Foggy | Foggy | Foggy | Clear | Clear | Clear | Clear |
| | Foggy | Foggy | Foggy | Clear | Clear | Clear | Clear |
| | Foggy | Foggy | Foggy | Clear | Clear | Clear | Clear |

Result: MIC- **0.225-0.55mM**

### Example 3

### Synthesis of "para position-PDEA", which is (S)-4-pyridylthio cysteamine.

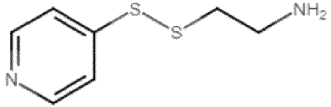

2-Aminoethanethiol hydrochloride (2.00 g, 17.6 mmol) was dissolved in methanol (3 ml) and acetic acid (1 ml). To the solution was added 4,4'-dithiodipyridine (5.00 g, 22.7 mmol) dissolved in methanol (20 ml). The mixture was stirred at 22°C overnight and then poured into dry diethyl ether (200 ml). A white precipitate formed that was collected by filtration.

Different concentrations of this compound in Mueller Hinton medium were prepared. Bacteria were inoculated to a concentration of 20 000 CFU/ml and incubated at 37°C for 24 hours. Minimum inhibitory concentrations (MICs) were determined by analyzing visible growth, i.e., readouts were "Clear" and "Foggy".

### Escherichia coli CFT073

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Clear | Clear | Clear | Clear |
| | Foggy | Foggy | Foggy | Foggy | Foggy | Clear | Clear | Clear | Clear |
| | Foggy | Foggy | Foggy | Foggy | Foggy | Clear | Clear | Clear | Clear |

Result: MIC- **0.2-0.4 mM**

### Klebsiella pneumoniae AO15200

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Clear | Clear | Clear | Clear |
| | Foggy | Foggy | Foggy | Foggy | Foggy | Clear | Clear | Clear | Clear |
| | Foggy | Foggy | Foggy | Foggy | Foggy | Clear | Clear | Clear | Clear |

Result: MIC- MIC- **0.2-0.4 mM**

### Pseudomonas aeruginosa PA02

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Foggy | Clear | Clear | Clear |
| | Foggy | Foggy | Foggy | Foggy | Foggy | Foggy | Clear | Clear | Clear |
| | Foggy | Foggy | Foggy | Foggy | Foggy | Foggy | Clear | Clear | Clear |

Result: MIC- **0.4- 0.8 mM**

### Staphylococcus aureus B5381

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Clear | Clear | Clear | Clear |
| | Foggy | Foggy | Foggy | Foggy | Foggy | Clear | Clear | Clear | Clear |
| | Foggy | Foggy | Foggy | Foggy | Foggy | Clear | Clear | Clear | Clear |

Result: MIC- MIC- **0.2-0.4 mM**

### Staphylococcus epidermidis Se19 (clinical isolate, Jan Ingemar Falk)

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Clear | Clear | Clear | Clear | Clear |
| | Foggy | Foggy | Foggy | Foggy | Clear | Clear | Clear | Clear | Clear |
| | Foggy | Foggy | Foggy | Foggy | Clear | Clear | Clear | Clear | Clear |

Result: MIC- **0.1-0.2 mM**

### Enterococcus faecalis #28 (KI, clinical isolate).

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Clear | Clear | Clear | Clear |
| | Foggy | Foggy | Foggy | Foggy | Foggy | Clear | Clear | Clear | Clear |
| | Foggy | Foggy | Foggy | Foggy | Foggy | Clear | Clear | Clear | Clear |

Result: MIC- **0.2-0.4 mM**

### Enterococcus faecium #11 (KI, clinical isolate)

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Clear | Clear | Clear | Clear | Clear |
| | Foggy | Foggy | Foggy | Foggy | Clear | Clear | Clear | Clear | Clear |
| | Foggy | Foggy | Foggy | Foggy | Clear | Clear | Clear | Clear | Clear |

Result: MIC- **0.1-0.2 mM**

Different concentrations of the Para position-PDEA" is (S)-4-pyridylthio cysteamine were also prepared in Yeast extract Peptone Dextrose (YPD) broth. Candida albicans were inoculated to a concentration of 20 000 CFU/ml and incubated at 37°C for 24 hours. Readouts were "Clear" and "Foggy".

### Candida albicans

| Conc., mM | 0 | 0.01 | 0.05 | 0.1 | 0.2 | 0.4 | 0.8 | 1.6 | 3.2 |
|---|---|---|---|---|---|---|---|---|---|
| | Foggy | Foggy | Foggy | Foggy | Foggy | Foggy | Foggy | Foggy | Clear |
| | Foggy | Foggy | Foggy | Foggy | Foggy | Foggy | Foggy | Foggy | Clear |
| | Foggy | Foggy | Foggy | Foggy | Foggy | Foggy | Foggy | Foggy | Clear |

Result: MIC - **1.6-3.2 mM**

### Example 4

### "Carboxylic acid PDEA" is 3-(2-Pyridyldithio)propanoic acid (CAS: 68617-64-1).

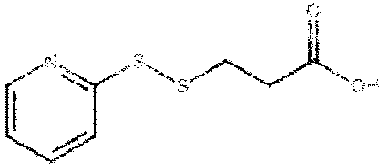

Different concentrations of this compound were prepared in Mueller Hinton broth. Bacteria were inoculated to a concentration of 20 000 CFU/ml and incubated at 37°C for 24 hours. Minimum inhibitory concentrations (MICs) were determined by analyzing visible growth, i.e., readouts were "Clear" and "Foggy".

### Escherichia coli CFT073

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Foggy | Foggy | Foggy | Clear |
| | Foggy | Foggy | Foggy | Foggy | Foggy | Foggy | Foggy | Clear |
| | Foggy | Foggy | Foggy | Foggy | Foggy | Foggy | Foggy | Clear |

Result: MIC- **2-4 mM**

### Klebsiella pneumoniae AO15200

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Foggy | Foggy | Foggy | Foggy |
| | Foggy | Foggy | Foggy | Foggy | Foggy | Foggy | Foggy | Foggy |
| | Foggy | Foggy | Foggy | Foggy | Foggy | Foggy | Foggy | Foggy |

| Conc., mM | 0 | 0.8 | 0.1.6 | 3.2 | 6.4 | 12.8 |
|---|---|---|---|---|---|---|
| | Foggy | Foggy | Foggy | Foggy | Clear | Clear |
| | Foggy | Foggy | Foggy | Foggy | Clear | Clear |
| | Foggy | Foggy | Foggy | Foggy | Clear | Clear |

Result: MIC- **3.2- 6.4mM**

### Pseudomonas aeruginosa PA02

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Foggy | Foggy | Foggy | Foggy |
| | Foggy | Foggy | Foggy | Foggy | Foggy | Foggy | Foggy | Foggy |
| | Foggy | Foggy | Foggy | Foggy | Foggy | Foggy | Foggy | Foggy |

| Conc., mM | 0 | 0.8 | 0.1.6 | 3.2 | 6.4 | 12.8 |
|---|---|---|---|---|---|---|
| | Foggy | Foggy | Foggy | Foggy | Foggy | Clear |
| | Foggy | Foggy | Foggy | Foggy | Foggy | Clear |
| | Foggy | Foggy | Foggy | Foggy | Foggy | Clear |

Result: MIC- **6.4-12.8mM**

### Staphylococcus aureus B5381

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Foggy | Clear | Clear | Clear |
| | Foggy | Foggy | Foggy | Foggy | Foggy | Clear | Clear | Clear |
| | Foggy | Foggy | Foggy | Foggy | Foggy | Clear | Clear | Clear |

Result: MIC- **0.5-1 mM**

### Staphylococcus epidermidis Se19 (clinical isolate, Jan Ingemar Falk)

| Conc., mM | 0 | 0.05 | 0.1 | 0.25 | 0.5 | 1 | 2 | 4 |
|---|---|---|---|---|---|---|---|---|
| | Foggy | Foggy | Foggy | Clear | Clear | Clear | Clear | Clear |
| | Foggy | Foggy | Foggy | Clear | Clear | Clear | Clear | Clear |
| | Foggy | Foggy | Foggy | Clear | Clear | Clear | Clear | Clear |

Result: MIC- **0.1-0.25 mM**

### Enterococcus faecalis #28 (KI, clinical isolate).

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Clear | Clear | Clear | Clear |
| | Foggy | Foggy | Foggy | Foggy | Clear | Clear | Clear | Clear |
| | Foggy | Foggy | Foggy | Foggy | Clear | Clear | Clear | Clear |

Result: MIC- **0.25-0.5 mM**

### Enterococcus faecium #11 (KI, clinical isolate)

| Conc., mM | 0 | 0.05 | 0.1 | 0.25 | 0.5 | 1 | 2 | 4 |
|---|---|---|---|---|---|---|---|---|
| | Foggy | Foggy | Foggy | Clear | Clear | Clear | Clear | Clear |
| | Foggy | Foggy | Foggy | Clear | Clear | Clear | Clear | Clear |
| | Foggy | Foggy | Foggy | Clear | Clear | Clear | Clear | Clear |

Result: MIC- **0.1-0.25 mM**

Different concentrations of "Carboxylic acid PDEA" is 3-(2-Pyridyldithio)propanoic acid (CAS: 68617-64-1) were also prepared in Yeast extract Peptone Dextrose (YPD) broth. Candida albicans were inoculated to a concentration of 20 000 CFU/ml and incubated at 37°C for 24 hours. Readouts were "Clear" and "Foggy".

### Candida albicans

| Conc., mM | 0 | 0.05 | 0.1 | 0.25 | 0.5 | 1 | 2 | 4 |
|---|---|---|---|---|---|---|---|---|
| | Foggy | Foggy | Foggy | Foggy | Foggy | Foggy | Clear | Clear |
| | Foggy | Foggy | Foggy | Foggy | Foggy | Foggy | Clear | Clear |
| | Foggy | Foggy | Foggy | Foggy | Foggy | Foggy | Clear | Clear |

Result: MIC- **1-2 mM**

### Example 5 - Cancer Cell Viability Assay

Two types of human uroepithelial cancer cells (T24 and 5637) were tested in an XTT assay. "T24" cells were cultured in McCoy's 5A (Modified) Medium, Catalog number: 16600082 from GIBCO. "5637" cells were cultured in RPMI 1640 Medium, Catalog number: 21875034 from GIBCO. Different concentrations of 2-(Pyridyldithio)ethylamine (hydrochloride) (PDEA) (CAS: 106139-15-5) : 12.5, 25, 50, 100 and 200 µM, were prepared in each cell culture medium.

### XTT ASSAY PROTOCOL (96 well assay):

Splitting ratio: 1:10
Day 1
1. 5 x 105 cells were seeded into 24-well plate overnight at 37-degree C in 5% CO₂.
Day 2
2. 80% confluent cells were treated with 200 µl medium with different concentrations of PDEA and the control.
3. After 24hrs, medium (MacCoy's 5a for T24, RPMI for 5637) was removed and cells were washed 2 times with 200 µl of pre-warmed PBS/well. 200 µl (96-well) of pre-prepared fresh medium containing XTT-menadione to wells were added (mentioned below).
4. Cells were incubated for at least 3 h at 37-degree C. Medium with XTT only in empty wells were served as background control.
5. The supernatant was transferred to 96-well plate and the concentration were measured at 450 nm and 690 nm. 690 nm was used as a background measurement and subtracted from 450 nm value.

### XTT-menadione solution preparation

Menadione (172.18 g/mol, light sensitive): dissolve 1 mg/ml in absolute ethanol. XTT: 1mg/ml dissolve in PBS, 0.2 µm filtrate sterilised, store at -20°C (Sigma X4626). Pre-warm to 37°C before use.

Prepare solution: Add 2.15 µl of menadione/ ml (final concentration 12.5 µM 0 2.15mg/l) to 1 ml of XTT directly before use. Prepare XTT-menadione solution equal to 20% of the culture medium volume to be tested.

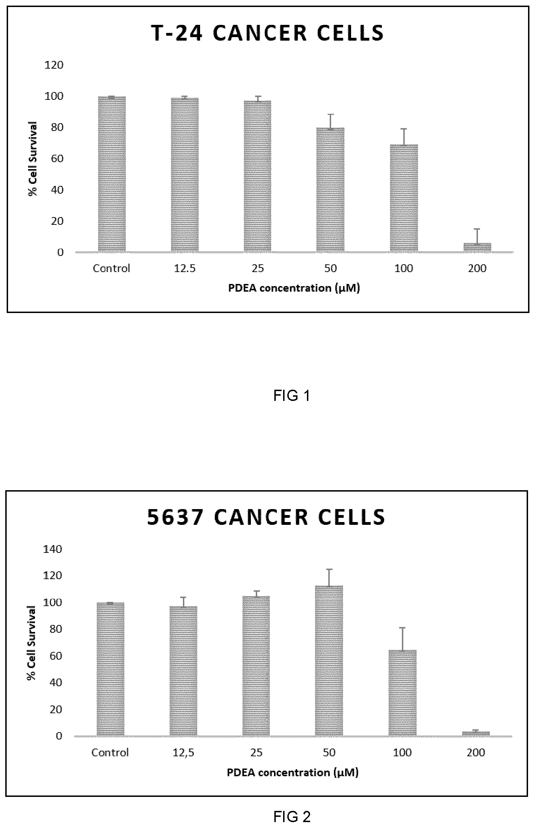

Results are shown below and represented graphically in Figures 1 and 2. "Blank" is cell culture medium only.

### T-24 Cells (triplicate)

| **Conc., µM** | Absorbance unit | Absorbance unit - Blank | **% cell survival** | Absorbance unit | Absorbance unit - Blank | **% cell survival** | Absorbance unit | Absorbance unit - Blank | **% cell survival** |
|---|---|---|---|---|---|---|---|---|---|
| Blank | 0.201 | | | 0.176 | | | 0.198 | | |
| Control | 1.639 | 1.438 | 100 | 1.68 | 1.504 | 100 | 1.886 | 1.688 | 100 |
| 12.5 | 1.63 | 1.429 | 99.37413 | 1.659 | 1.483 | 98.60372 | 1.884 | 1.686 | 99.88152 |
| 25 | 1.641 | 1.44 | 100.1391 | 1.605 | 1.429 | 95.0133 | 1.849 | 1.651 | 97.80806 |
| 50 | 1.3 | 1.099 | 76.42559 | 1.284 | 1.108 | 73.67021 | 1.711 | 1.513 | 89.6327 |
| 100 | 1.14 | 0.939 | 65.29903 | 1.387 | 1.211 | 80.51862 | 1.246 | 1.048 | 62.08531 |
| 200 | 0.439 | 0.238 | 16.55076 | 0.193 | 0.017 | 1.130319 | 0.197 | -0.001 | -0.05924 |

| | | | | | | | | | |
|---|---|---|---|---|---|---|---|---|---|
| Result: The cell viability of "T-24" cancer cells is being reduced by 10-26% at 50 µM, 20-28% at 100 µM, and 83-100% at 200 µM concentration of PDEA respectively. | | | | | | | | | |

### 5637 Cells (triplicate)

| **Conc., µM** | Absorbance unit | Absorbance unit - Blank | **% cell survival** | Absorbance unit | Absorbance unit - Blank | **% cell survival** | Absorbance unit | Absorbance unit - Blank | **% cell survival** |
|---|---|---|---|---|---|---|---|---|---|
| Blank | 0.157 | | | 0.155 | | | 0.148 | | |
| Control | 1.181 | 1.024 | 100 | 1.261 | 1.106 | 100 | 1.212 | 1.064 | 100 |
| 12.5 | 1.229 | 1.072 | 104.6875 | 1.224 | 1.069 | 96.65461 | 1.119 | 0.971 | 91.2594 |
| 25 | 1.274 | 1.117 | 109.082 | 1.305 | 1.15 | 103.9783 | 1.236 | 1.088 | 102.2556 |
| 50 | 1.335 | 1.178 | 115.0391 | 1.256 | 1.101 | 99.54792 | 1.463 | 1.315 | 123.5902 |
| 100 | 1.011 | 0.854 | 83.39844 | 0.739 | 0.584 | 52.80289 | 0.759 | 0.611 | 57.42481 |
| 200 | 0.194 | 0.037 | 3.613281 | 0.193 | 0.038 | 3.435805 | 0.199 | 0.051 | 4.793233 |

| | | | | | | | | | |
|---|---|---|---|---|---|---|---|---|---|
| Result: The cell viability of "5637" cancer cells is being reduced by 17-47% at 100 µM and 95-100% at 200 µM concentration of PDEA respectively. | | | | | | | | | |

### Example 6: Combination Therapy

Different concentrations of Fradiomycin in Mueller Hinton were prepared in 3 different concentrations of PDEA. E. coli and S. aureus were inoculated (10⁵) and were incubated overnight.

Readouts were "Clear" and "Foggy".

### E. coli

### Fradiomycin with no PDEA.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Foggy |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Foggy | Foggy | Foggy | Foggy | Foggy | Foggy | Foggy |
| Foggy | Foggy | Foggy | Foggy | Foggy | Foggy | Foggy |
| Foggy | Foggy | Foggy | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.0125mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Foggy | Foggy | Foggy | Foggy | Foggy | Foggy |
| Clear | Foggy | Foggy | Foggy | Foggy | Foggy | Foggy |
| Clear | Foggy | Foggy | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.025mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Foggy | Foggy | Foggy | Foggy |
| Clear | Clear | Foggy | Foggy | Foggy | Foggy | Foggy |
| Clear | Clear | Foggy | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.05mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Clear | Clear | Clear | Clear |
| Clear | Clear | Clear | Clear | Clear | Clear | Clear |
| Clear | Clear | Clear | Clear | Clear | Clear | Clear |

| | | | | | | |
|---|---|---|---|---|---|---|
| *Starting concentration of fradiomycin is 0.28mM | | | | | | |

### S. aureus

### Fradiomycin with no PDEA.

| Conc. µq/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Foggy | Foggy | Foggy | Foggy | Foggy | Foggy | Foggy |
| Foggy | Foggy | Foggy | Foggy | Foggy | Foggy | Foggy |
| Foggy | Foggy | Foggy | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.0125mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Foggy | Foggy | Foggy | Foggy | Foggy | Foggy |
| Clear | Clear | Foggy | Foggy | Foggy | Foggy | Foggy |
| Clear | Foggy | Foggy | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.025mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Foggy | Foggy | Foggy | Foggy |
| Clear | Clear | Foggy | Foggy | Foggy | Foggy | Foggy |
| Clear | Clear | Clear | Foggy | Foggy | Foggy | Foggy |

| | | | | | | |
|---|---|---|---|---|---|---|
| *Starting concentration of fradiomycin is 0.28mM | | | | | | |

### Fradiomycin with PDEA 0.05mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Clear | Foggy | Foggy | Foggy |
| Clear | Clear | Clear | Clear | Foggy | Foggy | Foggy |
| Clear | Clear | Clear | Clear | Clear | Foggy | Foggy |

| | | | | | | |
|---|---|---|---|---|---|---|
| *Starting concentration of fradiomycin is 0.28mM | | | | | | |

### Example 7: Combination Therapy

Different concentrations of Gentamycin in Mueller Hinton were prepared in 3 different concentrations of PDEA. E. coli and S. aureus were inoculated (10⁵) and were incubated O/N.

Readouts were "Clear" and "Foggy".

### E. coli

### Gentamycin with no PDEA.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Foggy | Foggy | Foggy | Foggy | Foggy | Foggy | Foggy |
| Foggy | Foggy | Foggy | Foggy | Foggy | Foggy | Foggy |
| Foggy | Foggy | Foggy | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.0125mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Foggy | Foggy | Foggy | Foggy | Foggy | Foggy |
| Clear | Foggy | Foggy | Foggy | Foggy | Foggy | Foggy |
| Clear | Foggy | Foggy | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.025mM.

| Conc. µq/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Foggy | Foggy | Foggy | Foggy | Foggy |
| Clear | Clear | Clear | Foggy | Foggy | Foggy | Foggy |
| Clear | Clear | Foggy | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.04mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Clear | Clear | Clear | Clear |
| Clear | Clear | Clear | Clear | Clear | Clear | Clear |
| Clear | Clear | Clear | Clear | Clear | Clear | Clear |

| | | | | | | |
|---|---|---|---|---|---|---|
| *Starting concentration of gentamycin is 0.44mM | | | | | | |

### S. aureus

### Gentamycin with no PDEA.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Foggy | Foggy | Foggy | Foggy | Foggy |
| Clear | Clear | Foggy | Foggy | Foggy | Foggy | Foggy |
| Clear | Clear | Foggy | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.0125mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Foggy | Foggy | Foggy | Foggy |
| Clear | Clear | Clear | Foggy | Foggy | Foggy | Foggy |
| Clear | Clear | Clear | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.025mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Clear | Foggy | Foggy | Foggy |
| Clear | Clear | Clear | Clear | Foggy | Foggy | Foggy |
| Clear | Clear | Clear | Foggy | Foggy | Foggy | Foggy |

| | | | | | | |
|---|---|---|---|---|---|---|
| *Starting concentration of gentamycin is 0.44mM | | | | | | |

### Gentamycin with PDEA 0.05mM.

| Conc. µg/ml | 256 | 128 | 64 | 32 | 16 | 8 | 4 | 2 | 1 |
|---|---|---|---|---|---|---|---|---|---|
| | Clear | Clear | Clear | Clear | Clear | Clear | Clear | Clear | Clear |
| | Clear | Clear | Clear | Clear | Clear | Clear | Clear | Clear | Clear |
| | Clear | Clear | Clear | Clear | Clear | Clear | Clear | Clear | Clear |

| 0.5 | 0.25 | 0.125 | 0.0625 | 0.03125 | 0.0156 | 0 |
|---|---|---|---|---|---|---|
| Clear | Clear | Clear | Clear | Clear | Foggy | Foggy |
| Clear | Clear | Clear | Clear | Clear | Foggy | Foggy |
| Clear | Clear | Clear | Clear | Clear | Foggy | Foggy |

| | | | | | | |
|---|---|---|---|---|---|---|
| *Starting concentration of gentamycin is 0.44mM | | | | | | |

## Claims

1. A disulfide compound selected from the group consisting of:
| **Compound** | **Structure** |
|---|---|
| 2-(Pyridyldithio)ethylamine (hydrochloride) (PDEA) | 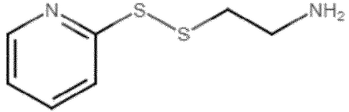 |
| PDEA (para position) | 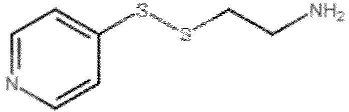 |
| PDEA analogue with quaternary amine (methyl) tail | 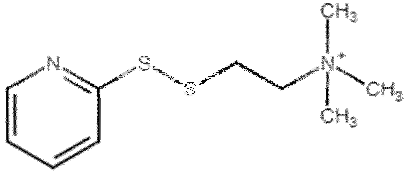 |
| 3-(2-Pyridyldithio)propanoic acid | 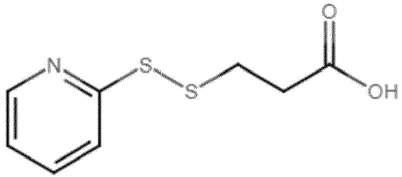 |
| PDEA analogue with carboxylic acid tail | |
and stereochemically isomeric forms thereof, and the pharmaceutically acceptable salts thereof for therapeutic use in a human or animal subject as an antimicrobial agent.

2. A disulfide compound for use according to claim 2, wherein the antimicrobial agent is an antibacterial agent.

3. Non-therapeutic use of a disulfide compound selected from the group consisting of:
| **Compound** | **Structure** |
|---|---|
| 2-(Pyridyldithio)ethylamine (hydrochloride) (PDEA) | 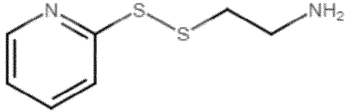 |
| PDEA (para position) | 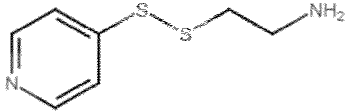 |
| PDEA analogue with quaternary amine (methyl) tail | 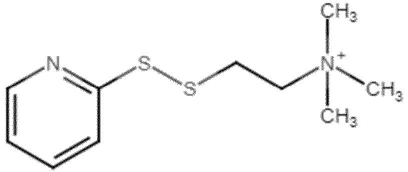 |
| 3-(2-Pyridyldithio)propanoic acid | 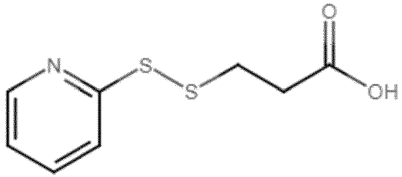 |
| PDEA analogue with carboxylic acid tail | |
and stereochemically isomeric forms thereof, and the pharmaceutically acceptable salts thereof as an antimicrobial agent.

4. A pharmaceutical composition comprising a disulfide compound selected from the group consisting of:
| **Compound** | **Structure** |
|---|---|
| 2-(Pyridyldithio)ethylamine (hydrochloride) (PDEA) | 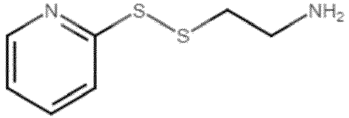 |
| PDEA (para position) | 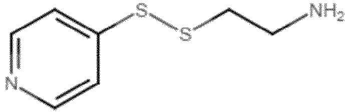 |
| PDEA analogue with quaternary amine (methyl) tail | 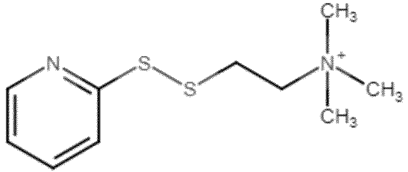 |
| 3-(2-Pyridyldithio)propanoic acid | 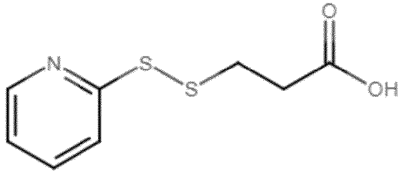 |
| PDEA analogue with carboxylic acid tail | |
and stereochemically isomeric forms thereof, and the pharmaceutically acceptable salts thereof; and one or more other antibiotics.

5. The pharmaceutical composition as claimed in claim 4 wherein said one or more other antibiotics is an aminoglycoside.

6. The pharmaceutical composition as claimed in claim 5 wherein the aminoglycoside is fradiomycin or gentamycin.

7. A composition as defined in any of claims 4 to 6 for use as a medicament.

8. A composition as defined in any of claims 4 to 6 for use as an antimicrobial agent.

9. The compound for use, the use, or the composition of any of claims 1-8, wherein the disulfide compound has the following structure:

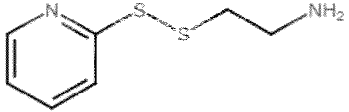

10. The compound for use, the use, or the composition of any of claims 1-8, wherein the disulfide compound has the following structure:

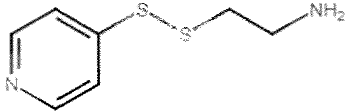

11. The compound for use, the use, or the composition of any of claims 1-8, wherein the disulfide compound has the following structure:

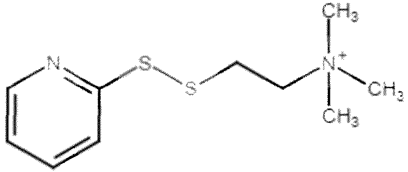

12. The compound for use, the use, or the composition of any of claims 1-8, wherein the disulfide compound has the following structure:

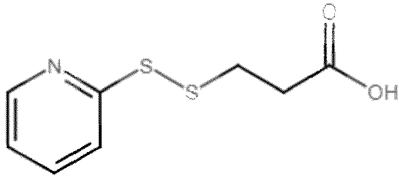

## Patentansprüche

1. Disulfidverbindung, ausgewählt aus der Gruppe bestehend aus:
| **Verbindung** | **Struktur** |
|---|---|
| 2-(Pyridyldithio)ethylamin (hydrochlorid) (PDEA) | 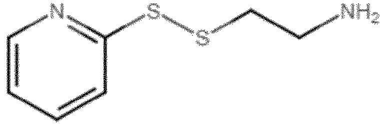 |
| PDEA (para-Position) | 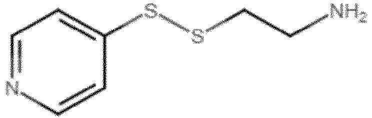 |
| PDEA-Analogon mit quaternärem Amin (Methyl) schwanz | 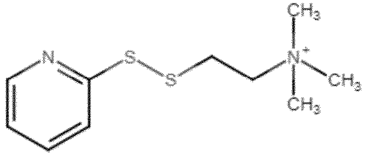 |
| 3-(2-Pyridyldithio)propan säure | 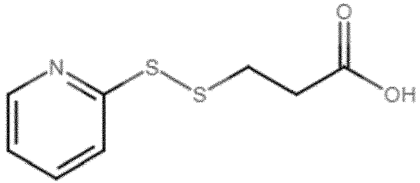 |
| PDEA-Analogon mit Carbonsäureschwanz | |
und stereochemisch isomeren Formen davon und die pharmazeutisch verträglichen Salze davon zur therapeutischen Verwendung bei einem Menschen oder Tier als antimikrobielles Mittel.

2. Disulfidverbindung zur Verwendung nach Anspruch 2, wobei das antimikrobielle Mittel ein antibakterielles Mittel ist.

3. Nicht-therapeutische Verwendung einer Disulfidverbindung, ausgewählt aus der Gruppe bestehend aus:
| **Verbindung** | **Struktur** |
|---|---|
| 2-(Pyridyldithio)ethylamin (hydrochlorid) (PDEA) | 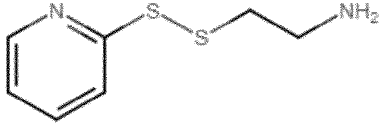 |
| PDEA (para-Position) | 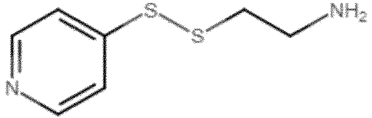 |
| PDEA-Analogon mit quaternärem Amin (Methyl) schwanz | 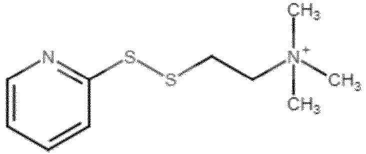 |
| 3-(2-Pyridyldithio)propan säure | 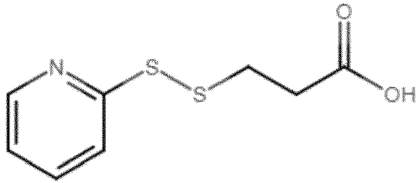 |
| PDEA-Analogon mit Carbonsäureschwanz | |
und stereochemisch isomeren Formen davon und die pharmazeutisch verträglichen Salze davon als antimikrobielles Mittel.

4. Pharmazeutische Zusammensetzung, umfassend eine Disulfidverbindung, ausgewählt aus der Gruppe bestehend aus:
| **Verbindung** | **Struktur** |
|---|---|
| 2-(Pyridyldithio)ethylamin (hydrochlorid) (PDEA) | 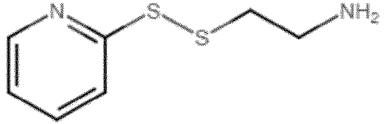 |
| PDEA (para-Position) | 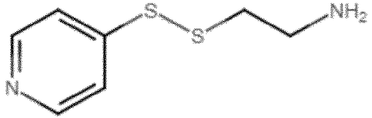 |
| PDEA-Analogon mit quaternärem Amin (Methyl) schwanz | 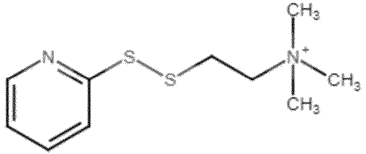 |
| 3-(2-Pyridyldithio)propan säure | 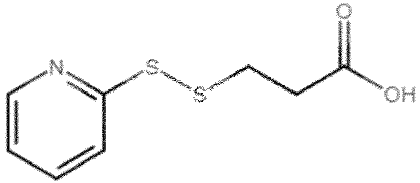 |
| PDEA-Analogon mit Carbonsäureschwanz | |
und stereochemisch isomere Formen davon und die pharmazeutisch verträglichen Salze davon;
und ein oder mehrere andere Antibiotika.

5. Pharmazeutische Zusammensetzung nach Anspruch 4, wobei das eine oder die mehreren anderen Antibiotika ein Aminoglycosid ist.

6. Pharmazeutische Zusammensetzung nach Anspruch 5, wobei das Aminoglycosid Fradiomycin oder Gentamycin ist.

7. Zusammensetzung nach einem der Ansprüche 4 bis 6 zur Verwendung als Medikament.

8. Zusammensetzung nach einem der Ansprüche 4 bis 6 zur Verwendung als antimikrobielles Mittel.

9. Verbindung zur Verwendung, die Verwendung oder die Zusammensetzung nach einem der Ansprüche 1 bis 8, wobei die Disulfidverbindung die folgende Struktur aufweist:

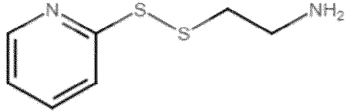

10. Verbindung zur Verwendung, die Verwendung oder die Zusammensetzung nach einem der Ansprüche 1 bis 8, wobei die Disulfidverbindung die folgende Struktur aufweist:

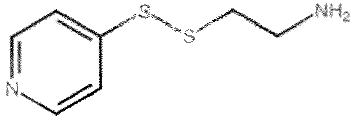

11. Verbindung zur Verwendung, die Verwendung oder die Zusammensetzung nach einem der Ansprüche 1 bis 8, wobei die Disulfidverbindung die folgende Struktur aufweist:

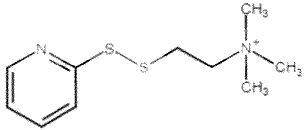

12. Verbindung zur Verwendung, die Verwendung oder die Zusammensetzung nach einem der Ansprüche 1 bis 8, wobei die Disulfidverbindung die folgende Struktur aufweist:

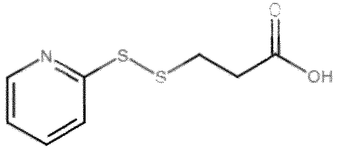

## Revendications

1. Composé disulfure sélectionné dans le groupe consistant en :
| **Composé** | **Structure** |
|---|---|
| 2-(pyridyldithio)éthylamine (chlorhydrate) (PDEA) | 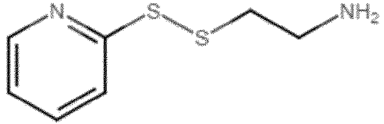 |
| PDEA (position para) | 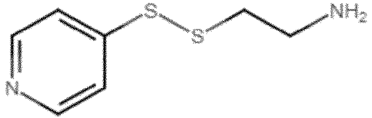 |
| Analogue de PDEA avec queue amine quaternaire (méthyle) | 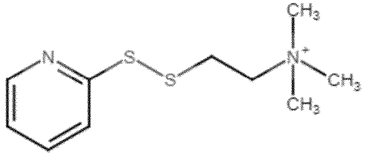 |
| Acide 3-(2-pyridyldithio) propanoïque Analogue de PDEA avec queue acide carboxylique | 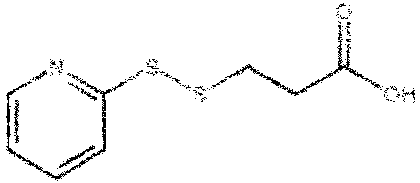 |
et des formes stéréochimiquement isomères de ceux-ci, et les sels pharmaceutiquement acceptables de ceux-ci pour utilisation thérapeutique chez un sujet humain ou animal en tant qu'agent antimicrobien.

2. Composé disulfure pour utilisation selon la revendication 2, dans lequel l'agent antimicrobien est un agent antibactérien.

3. Utilisation non thérapeutique d'un composé disulfure sélectionné dans le groupe consistant en :
| **Composé** | **Structure** |
|---|---|
| 2-(pyridyldithio)éthylamine (chlorhydrate) (PDEA) | 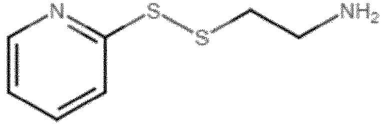 |
| | |
| PDEA (position para) | 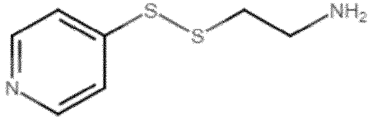 |
| Analogue de PDEA avec queue amine quaternaire (méthyle) | 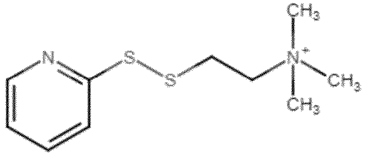 |
| Acide 3-(2-pyridyldithio) propanoïque Analogue de PDEA avec queue acide carboxylique | 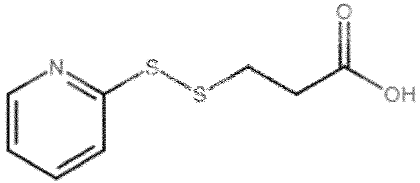 |
et des formes stéréochimiquement isomères de ceux-ci, et les sels pharmaceutiquement acceptables de ceux-ci en tant qu'agent antimicrobien.

4. Composition pharmaceutique comprenant un composé disulfure sélectionné dans le groupe consistant en :
| **Composé** | **Structure** |
|---|---|
| 2-(pyridyldithio)éthylamine (chlorhydrate) (PDEA) | 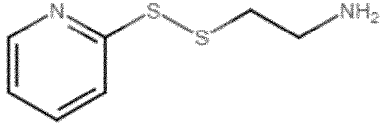 |
| PDEA (position para) | 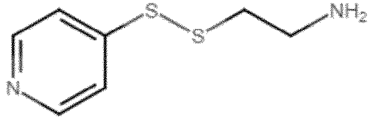 |
| Analogue de PDEA avec queue amine quaternaire (méthyle) | 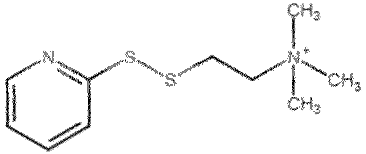 |
| Acide 3-(2-pyridyldithio) propanoïque Analogue de PDEA avec queue acide carboxylique | 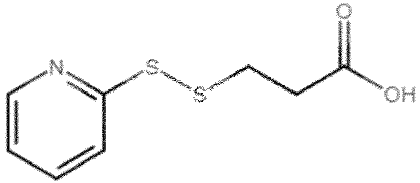 |
et des formes stéréochimiquement isomères de ceux-ci, et les sels pharmaceutiquement acceptables de ceux-ci ;
et un ou plusieurs autres antibiotiques.

5. Composition pharmaceutique selon la revendication 4, dans laquelle lesdits un ou plusieurs autres antibiotiques sont un aminoglycoside.

6. Composition pharmaceutique selon la revendication 5, dans laquelle l'aminoglycoside est la fradiomycine ou la gentamycine.

7. Composition telle que définie dans l'une quelconque des revendications 4 à 6, pour utilisation en tant que médicament.

8. Composition telle que définie dans l'une quelconque des revendications 4 à 6, pour utilisation en tant qu'agent antimicrobien.

9. Composé pour utilisation, utilisation ou composition selon l'une quelconque des revendications 1-8, dans lequel ou dans laquelle le composé disulfure présente la structure suivante :

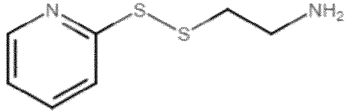

10. Composé pour utilisation, utilisation ou composition selon l'une quelconque des revendications 1-8, dans lequel ou dans laquelle le composé disulfure présente la structure suivante :

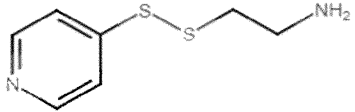

11. Composé pour utilisation, utilisation ou composition selon l'une quelconque des revendications 1-8, dans lequel ou dans laquelle le composé disulfure présente la structure suivante :

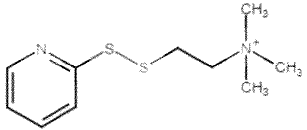

12. Composé pour utilisation, utilisation ou composition selon l'une quelconque des revendications 1-8, dans lequel ou dans laquelle le composé disulfure présente la structure suivante :
